# EUROPEAN PATENT APPLICATION

(11) **EP 1 602 733 A1**
(43) Date of publication of application: **07.12.2005**
(21) Application number: 04076618.0
(22) Date of filing: 02.06.2004
(51) Int. Cl.: C12Q 1/68

(54) **Detection of target nucleotide sequences using an asymmetric oligonucleotide ligation assay**

(71) Applicant: KEYGENE N.V., 6708 PW Wageningen (NL)
(72) Inventor: Hogers, René Cornelis Josephus, 6715 GR Ede (NL)
(74) Representative: de Lang, Robbert-Jan

(57) **Abstract**

The invention relates to a method for the detection target nucleic acid sequences based on ligation of a first and a second probe that are hybridized adjacent on a target nucleic acid sequence, removal of any unligated probes, preferably by exonuclease treatment, amplification of a portion of the second probe that does not contain the target specific section to provide amplicons and detection of said amplicons. The second probe comprises a second target binding section, one or two primer binding sequences and an identifier. The first probe preferably does not comprise a primer binding sequence, preferably consists of a first target binding section and is preferably exonuclease resistant.

SEQUENCE LISTING

<110>  Keygene NV

<120>  Detection of target nucleotide sequences using an asymmetric
oligonucleotide ligation assay.

<130>  P037

<160>  72

<170>  PatentIn version 3.1

<210>  1
<211>  16
<212>  DNA
<213>  oligonucleotide primer

<400>  1
gatgagtcct gagtaa                                                    16

<210>  2
<211>  16
<212>  DNA
<213>  oligonucleotide primer

<400>  2
gactgcgtac caattc                                                    16

<210>  3
<211>  472
<212>  DNA
<213>  Lycopersicon esculentum

<220>
<221>  misc_feature
<223>  W= A or T; M= A or C; R= A or G; Y= C or T; K= G or T; S= G or C;
        H= A, C or T; B= C, G or T; V= A, C or G; D= A, G or T; N= A, C,
        G or T

<400>  3
tatccactca ggtctccgca agccagaaat gggatataca ccttgttacg accytcaagc     60

catccactac tgcaatctgt catgtcacag atgttcggaa gataatgtat aagtacaact    120

atatagtcgg awttgcatct agtctagcat tcggaaaatg gaagccatgc tacttctagc    180

ataaaaaaca gcagctagaa atcgtaactc caatgatacg aggaagtatt cagagtttag    240

agtgawgtac aatgcaattt agagaacaag  catctgcaca tcraagttac ctaggtcctc    300

agcgcctgat ggacttccaa cttgttcaag aaggcgataa aggtctttct cattgaatcc    360

ttcaggtgga gagtagtttt cacaaactgc aaatgcctct gcacagcgga aagattgaat    420

tagatttatg ttatatagcc attctagtct tgctttaatg gatctttctc ga            472

<210>  4
<211>  222

<212>  DNA
<213>  Lycopersicon esculentum

<220>
<221>  misc_feature
<223>  W= A or T; M= A or C; R= A or G; Y= C or T; K= G or T; S= G or C;
       H= A, C or T; B= C, G or T; V= A, C or G; D= A, G or T; N= A, C,
       G or T


<400>  4
ccacagtttc atgctgcacc tacatgtgta agcaactatc atagcaagtc tcggaacaat     60

tggtaggaaa aaatcmykta aggatatgaa acatactgty ctttcttcat ctgagtctgy    120

agagttaatt tttaactctt gggataaatg caaagawtta gacatggakg agtycttaac    180

acgtccagac aagaggcgta acacaggtac accttttctc ga                       222


<210>  5
<211>  133
<212>  DNA
<213>  Lycopersicon esculentum

<220>
<221>  misc_feature
<223>  W= A or T; M= A or C; R= A or G; Y= C or T; K= G or T; S= G or C;
       H= A, C or T; B= C, G or T; V= A , C or G; D= A, G or T; N= A, C,
       G or T


<400>  5
ttgtgcttga tgaattgtag gtccagtgca ggtttgcttc taaaacaggg agcactttgc     60

aagtggtgaa agttctatta gctgggaaag tgtagtttga gcagttttga gctgarttaa    120

caagaaaaat cga                                                       133


<210>  6
<211>  249
<212>  DNA
<213>  Lycopersicon esculentum

<220>
<221>  misc_feature
<223>  W= A or T; M= A or C; R= A or G; Y= C or T; K= G or T; S= G or C;
       H= A, C or T; B= C, G or T; V= A, C or G; D= A, G or T; N= A , C,
       G or T


<400>  6
ccgccactgg gtaattgagt ttcatattga tggttttgtt tttgttracg cttcttcctt     60

gttgagaggg ttcaatggag agattctatc tcgtcctcca ttagttgaag ctattgcctt    120

tgatcctatc ctttcaaagg ycaagatgat tgcagataat tggaatccat taaccaatga    180

ttctacggaa aatttattcc ctcactggag gagatgggca gagataaata tgagattttg    240

tgatgacat                                                            249

<210>  7
<211>  284
<212>  DNA
<213>  Lycopersicon esculentum

<220>
<221>  misc_feature
<223>  W= A or T; M= A or C; R= A or G; Y= C or T; K= G or T; S= G or C;
       H= A, C or T; B= C, G or T; V= A, C or G; D= A, G or T; N= A, C,
       G or T

<400>  7
tcgagtaagg cggatggata tggaacaagc catttcaagg agcaatttcc caggattttc     60

agctttgcaa cagcagaagt gtayctctg c agagatagat cataaccttt ggaaaggtgt    120

agtaattgtc aaagggagga atgagccagg aaactgatag actatgttgc gaaaataagc    180

tatacttcac taaaaaaagg ctagacgttt gagaaatgaa gcaagaacta acacctctca    240

ccaattgcat cattttctta gttcagttga tgtgatgagc ttgt                      284

<210>  8
<211>  320
<212>  DNA
<213>  Lycopersicon esculentum

<220>
<221>  misc_feature
<223>  W= A or T; M= A or C; R= A or G; Y= C or T; K= G or T; S= G or C;
       H= A, C or T; B= C, G or T; V= A, C or G; D= A, G or T; N= A, C,
       G or T

<400>  8
tcgatatccw ctcttgtttg ttgcaggagc wgaactataa attgcttgca ggaaccttga     60

catatgcttt ctgttgagac ttgaatcacc agcatggatt tgaatgcctt gccacagcca    120

gaggatgacg aygagatttt tggacaacaa ttagaagatg aaccacaaga acctatttta    180

cgtagtgatg agcstgca ga ttatgtcacg agtgctgtag agatttcacg tcgcgtatgt    240

ttctgcttat actgctcgct gtatcaacta ttgaacygta ctactacttg arcttgctcg    300

tttattggat atttcttttt                                                 320

<210>  9
<211>  191
<212>  DNA
<213>  Lycopersicon e sculentum

<220>
<221>  misc_feature
<223>  W= A or T; M= A or C; R= A or G; Y= C or T; K= G or T; S= G or C;
       H= A, C or T; B= C, G or T; V= A, C or G; D= A, G or T; N= A, C,
       G or T

<400> 9
gaattcacac tasgttcgat gaaattgaaa cgttctcttt ctg aagaaka tacacaagaa 60

aaaatcttat agtcctcaac aatattcttc ttcgtaacag aaaacacgga agaaaatctc 120

ttctgaaaat ccctataatc actggctgga acttctccsa actctcaatt tttcaacctt 180

ctctatgtta a 191


<210> 10
<211> 279
<212> DNA
<213> Lycopersicon esculentum

<220>
<221> misc_feature
<223> W= A or T; M= A or C; R= A or G; Y= C or T; K= G or T; S= G or C;
H= A, C or T; B= C, G or T; V= A, C or G; D= A, G or T; N= A, C,
G or T


<400> 10
ctgcagaadt actgtttgtt caggacttac taaatatcct aaacaaaatt gatgatagag 60

ccaataatgt atgcatgatt ggcggtccrt tcttttgtta tagcaagagc ttgaagctaa 120

ttttgtttgt cataatggcc gcactaattg tttattatct cagaatgaac aaaaagaagc 180

aagtcagaag ctttstactc tatactgaac aactttggaa ttggaactat gtacttatct 240

agccacgcct catagatctt tgtggtttag gagtgttaa 279


<210> 11
<211> 336
<212> DNA
<213> Lycopersicon esculentum

<220>
<221> misc_feature
<223> W= A or T; M= A or C; R= A or G; Y= C or T; K= G or T; S= G or C;
H= A, C or T; B= C, G or T; V= A, C or G; D= A, G or T; N= A, C,
G or T


<400> 11
gaattcacaa tgaaaaakgk dgtaaaaaca cgaaatcaat caagcatgca agagataatg 60

ttgtccatcc agttgttgtt gatgtttcgg tattgtatgt gtgttgggag gagt tatctg 120

grcagcaagt cgaggtttga acgtcaaaaa ggtatgggtt gtcttctctc tttgtccctt 180

ttcgaagaga cccctaaggt tcagacgaat ctattccaaa aactagggtt gttccttgtt 240

gcatctcctt ktcacaagct cccatcgcat cataagtagg gtatgtttga tggtagaatt 300

tacggatgta atttactt tt gaaatgatta tgttaa 336


<210> 12
<211> 373
<212> DNA

<213>  Lycopersicon esculentum

<220>
<221>  misc_feature
<223>  W= A or T; M= A or C; R= A or G; Y= C or T; K= G or T; S= G or C;
       H= A, C or T; B= C, G or T; V = A, C or G; D= A, G or T; N= A, C,
       G or T


<400>  12
agagagacga gagctcgact agtgatagtg ttatgtgcaa cagttgaata gaaagatgya     60

cacgagcctc ggatcaatgg cagggaaaga ggcgtggtgc tacgaaccat aaaggcaagg    120

ttgagctttc ctttacagag tacatcgcct attccatact c cgctgatac tctttgataa    180

atcaaaatct gtggtgatct cgtagttctt ggggatccca gccaaaacca ccttcgaggt    240

tcaacacaac atagacagta tggcagaata tcaagacaat gactgctcga aactgctgat    300

ggcattatgt gcaaccgttg aatagagaga tgtacacgag tctcggatca atggcaggaa    360

aagagagtgc ttg                                                       373


<210>  13
<211>  45
<212>  DNA
<213>  oligonucleotide probe

<400>  13
gtacaatgca atttagagaa caagcccggg cggcccgggc gcggc                     45


<210>  14
<211>  43
<212>  DNA
<213>  oligonucleotide probe

<400>  14
cttaacacgt ccagacaaga ggccgcgggc gcgcggcggg cgg                       43


<210>  15
<211>  45
<212>  DNA
<213>  oligonucleotide probe

<400>  15
ttaacaagaa aaatcggtca ggactcgcgg cgcccgcggc gcggg                     45


<210>  16
<211>  43
<212>  DNA
<213>  oligonucleotide probe

<400>  16
acgcttcttc cttgttgaga gggcgccggc cgggcccgcc ggc                       43


<210>  17
<211>  44

<212>  DNA
<213>  oligonucleotide probe

<400>  17
ctctgcagag atagatcata acctggcccg cgcgcccggc  ggcg                    44


<210>  18
<211>  45
<212>  DNA
<213>  oligonucleotide probe

<400>  18
gaactataaa ttgcttgcag gaaccgggcg gcccggcccg cccgg                    45


<210>  19
<211>  46
<212>  DNA
<213>  oligonucleotide probe

<400>  19
aactctcaat ttttcaacct tctctacgcg ccgggccgcg gccggc                   46


<210>  20
<211>  46
<212>  DNA
<213>  oligonucleotide probe

<400>  20
ttcttttgtt atagcaagag cttgaagccg gccggccgcg cgcggg                   46


<210>  21
<211>  42
<212>  DNA
<213>  oligonucleotide probe

<400>  21
tcacaagctc ccatcgcatc atcggcgcgc gggccgcgcg cc                       42


<210>  22
<211>  40
<212>  DNA
<213>  oligonucleotide probe

<400>  22
acacgagcct cggatcaatg cggcccgccc ggcggccgcc                          40


<210>  23
<211>  25
<212>  DNA
<213>  oligonucleotide probe

<400>  23
gtacaatgca atttagagaa caagc                                          25


<210>  24
<211>  23

<212>  DNA
<213>  oligonucleotide probe

<400>  24
cttaacacgt ccagacaaga ggc                                              23


<210>  25
<211>  25
<212>  DNA
<213>  oligonucleotide probe

<400>  25
ttaacaagaa aaatcggtca ggact                                            25


<210>  26
<211>  23
<212>  DNA
<213>  oligonucleotide probe

<400>  26
acgcttcttc cttgttgaga ggg                                              23


<210>  27
<211>  24
<212>  DNA
<213>  oligonucleotide probe

<400>  27
ctctgcagag atagatcata acct                                             24


<210>  28
<211>  25
<212>  DNA
<213>  oligonucleotide probe

<400>  28
gaactataaa ttgcttgcag gaacc                                            25


<210>  29
<211>  26
<212>  DNA
<213>  oligonucleotide probe

<400>  29
aactctcaat ttttcaacct tctcta                                           26


<210>  30
<211>  26
<212>  DNA
<213>  oligonucleotide probe

<400>  30
ttcttttgtt atagcaagag cttgaa                                           26


<210>  31
<211>  22

<212>  DNA
<213>  oligonucleotide probe

<400>  31
tcacaagctc ccatcgcatc at                                                22


<210>  32
<211>  20
<212>  DNA
<213>  oligonucleotide probe

<400>  32
acacgagcct cggatcaatg                                                   20


<210>  33
<211>  45
<212>  DNA
<213>  oligonucleotide probe

<400>  33
gtacaatgca atttagagaa caagcccggg cggcccgggc gcggc                       45


<210>  34
<211>  43
<212>  DNA
<213>  oligonucleotide probe

<400>  34
cttaacacgt ccagacaaga ggccgcgggc gcgcggcggg cgg                         43


<210>  35
<211>  45
<212>  DNA
<213>  oligonuc leotide probe

<400>  35
ttaacaagaa aaatcggtca ggactcgcgg cgcccgcggc gcggg                       45


<210>  36
<211>  43
<212>  DNA
<213>  oligonucleotide probe

<400>  36
acgcttcttc cttgttgaga gggcgccggc cgggcccgcc ggc                         43


<210>  37
<211>  44
<212>  DNA
<213>  oligonucleotide probe

<400>  37
ctctgcagag atagatcata acctggcccg cgcgcccggc ggcg                        44


<210>  38
<211>  45

<212>  DNA
<213>  oligonucleotide probe

<400>  38
gaactataaa ttgcttgcag gaaccgggcg gcccggcccg cccgg                    45


<210>  39
<211>  46
<212>  DNA
<213>  oligonucleotide probe

<400>  39
aactctcaat ttttcaacct tctctacgcg ccgggccgcg gccggc                   46


<210>  40
<211>  46
<212>  DNA
<213>  oligonucleotide probe

<400>  40
ttcttttgtt atagcaagag cttgaagccg gccggccgcg cgcggg                   46


<210>  41
<211>  42
<212>  DNA
<213>  oligonucleotide probe

<400>  41
tcacaagctc ccatcgcatc atcggcgcgc gggccgcgcg cc                       42


<210>  42
<211>  40
<212>  DNA
<213>  oligonucleotide probe

<400>  42
acacgagcct cggatcaatg cggcccgccc ggcggccgcc                          40


<210>  43
<211>  25
<212>  DNA
<213>  oligonucleotide probe

<400>  43
gtacaatgca atttagagaa caagc                                          25


<210>  44
<211>  23
<212>  DNA
<213>  oligonucleotide probe

<400>  44
cttaacacgt ccagacaaga ggc                                            23


<210>  45
<211>  25

<212>  DNA
<213>  oligonucleotide probe

<400>  45
ttaacaagaa aaatcggtca ggact                                          25


<210>  46
<211>  23
<212>  DNA
<213>  oligonucleotide probe

<400>  46
acgcttcttc cttgttgaga ggg                                            23


<210>  47
<211>  24
<212>  DNA
<213>  oligonucleotide probe

<400>  47
ctctgcagag atagatcata acct                                           24


<210>  48
<211>  25
<212>  DNA
<213>  oligonucleotide probe

<400>  48
gaactataaa ttgcttgcag gaacc                                          25


<210>  49
<211>  26
<212>  DNA
<213>  oligonucleot ide probe

<400>  49
aactctcaat ttttcaacct tctcta                                         26


<210>  50
<211>  26
<212>  DNA
<213>  oligonucleotide probe

<400>  50
ttcttttgtt atagcaagag cttgaa                                         26


<210>  51
<211>  22
<212>  DNA
<213>  oligonucleotide probe

<400>  51
tcacaagctc ccatcgcatc at                                             22


<210>  52
<211>  20

<212>  DNA
<213>  oligonucleotide probe

<400>  52
acacgagcct cggatcaatg                                                    20


<210>  53
<211>  154
<212>  DNA
<213>  oligonucleotide probe

<400>  53
gccgcgcccg ggccgcccgg gatgagtcct gagtaacgac ttcagttcca ctggcctgtc      60

gtaaggtacg gcctgagtgg aagtaaattc aacgtgggtc tccggtaccg ggaattggta     120

cgcagtcctg gaagtattca gagtttagag tgaa                                 154


<210>  54
<211>  150
<212>  DNA
<213>  oligonucleotide probe

<400>  54
gccgcgcccg ggccgcccgg gatgagtcct gagtaacgac ttcagttcca ctggcctgtc      60

gtaaggtacg gcctgagtgg aagtaaattc aacgtgggtc tccgg taggg aattggtacg    120

cagtcggaag tattcagagt ttagagtgat                                      150


<210>  55
<211>  149
<212>  DNA
<213>  oligonucleotide probe

<400>  55
ccgcccgccg cgcgcccgcg gatgagtcct gagtaacgcc gtaaatctca gcacaagtca      60

tcagggttga tacgaaatcg ggcacaggct tagcgctcag aaacgggaat tggtacgcag     120

tccagcaaag aattagacat ggatgagtt                                       149


<210>  56
<211>  145
<212>  DNA
<213>  oligonucleotide probe

<400>  56
ccgcccgccg cgcgcccgcg gatgagtcct gagtaacgcc g taaatctca gcacaagtca     60

tcagggttga tacgaaatcg ggcacaggct tagcgctcag aagggaattg gtacgcagtc    120

ccaaagattt agacatggag gagtc                                           145


<210>  57
<211>  143
<212>  DNA
<213>  oligonucleotide probe

<400> 57
cccgcgccgc gggcgccgcg gatgagtcct gagtaacgct cctgcttgcc cgcaaaccca 60

ggcctgcaga tctcacggat tgcagtatct taccctaaag ggaattggta cgcagtccct 120

agtttgagca gttttgagct gaa 143

<210> 58
<211> 139
<212> DNA
<213> oligonucleotide probe

<400> 58
cccgcgccgc gggcgccgcg gatgagtcct gagtaacgct cctgcttgcc cgcaaaccca 60

ggcctgcaga tctcacggat tgcagtatct taccctaggg aattggtacg cagtctagtt 120

tgagcagttt tgagctgag 139

<210> 59
<211> 141
<212> DNA
<213> oligonucleotide probe

<400> 59
gccggcgggc ccggccggcg gatgagtcct gagtaacgtc caggctgaca gacagaacgc 60

tgtcttataa atcactcttt cattgcgcga cgccgggaat tggtacgcag tcccttcata 120

ttgatggttt tgtttttgtt a 141

<210> 60
<211> 153
<212> DNA
<213> oligonucleotide probe

<400> 60
gccggcgggc ccggccggcg gatgagtcct gagtaagatg agtcctgagt aacgtccagg 60

ctgacagaca gaacgctgtc ttataaatca ctctttcatt gcgcgacggg gaattggtac 12 0

gcagtcttca tattgatggt tttgtttttg ttg 153

<210> 61
<211> 149
<212> DNA
<213> oligonucleotide probe

<400> 61
cgccgccggg cgcgcgggcc gatgagtcct gagtaagatg agtcctgagt aacgacacgt 60

agctctacct tagtggtgcc tgccgcacgt aaggcctcat gtcctgggaa ttggtacgca 120

gtccaagctt tgcaacagca gaagtgtat 149

<210> 62
<211> 145

<212>  DNA
<213>  oligonucleotide probe

<400>  62
cgccgccggg cgcgcgggcc gatgagtcct gagtaagatg agtcctgagt aacgacacgt      60

agctctacct tagtggtgcc tgccgcacgt aaggcctcat gtcgggaatt ggtacgcagt     120

cagctttgca acagcagaag tgtac                                           145


<210>  63
<211>  143
<212>  DNA
<213>  oligonucleotide probe

<400>  63
ccgggcgggc cgggccgccc gatgag tcct gagtaagatg agtcctgagt aacggtgcta      60

gtaatctagc gtacgttgtt aaacatctcc tcacggtacg gggaattggt acgcagtcct     120

ctctcttgtt tgttgcagga gca                                             143


<210>  64
<211>  139
<212>  DNA
<213>  oligonucleotide probe

<400>  64
ccgggcgggc cgggccgccc gatgagtcct gagtaagatg agtcctgagt aacggtgcta      60

gtaatctagc gtacgttgtt aaacatctcc tcacggtagg gaattggtac gcagtccact     120

cttgtttgtt gcaggagct                                                  139


<210>  65
<211>  137
<212>  DNA
<213>  oligonucleotide probe

<400>  65
gccggccgcg gcccggcgcg gatgagtcct gagtaagatg agtcctgagt aacgagggtc      60

ccgccacgag ctgcttcatc atgtagaccc agttggggaa ttggtacgca gtccgatcac     120

tggctggaac ttctccc                                                    137


<210>  66
<211>  133
<212>  DNA
<213>  oligonucleotide probe

<400>  66
gccggccgcg gcccggcgcg gatgagtcct gagtaagatg agtcctgagt aacgagggtc      60

ccgccacgag ctgcttcatc atgtagaccc agtgggaatt ggtacgcagt catcactggc     120

tggaacttct ccg                                                        133

<210> 67
<211> 133
<212> DNA
<213> oligonucleotide probe

<400> 67
cccgcgcgcg gccggccggc gatgagtcct gagtaagatg agtcctgagt aacgctcgcg 60

ccgacagcgt cgcgagagca cctaaatgtc gggaattggt acgcagtccc atgtatgcat 120

gattggcggt cca 133

<210> 68
<211> 129
<212> DNA
<213> oligonucleotide probe

<400> 68
cccgcgcgcg gccggccggc gatgagtcct gagtaagatg agtcctgagt aacgctcgcg 60

ccgacagcgt cgcgagagca cctaaatggg gaattggtac gcagtcatgt atgcatgatt 120

ggcggtccg 129

<210> 69
<211> 129
<212> DNA
<213> oligonucleotide probe

<400> 69
ggcgcgcggc ccgcgcgccg gatgagtcct gagtaagatg agtcct gagt aacgctttct 60

gtacgtcccg ggcatagtga attctgggaa ttggtacgca gtcctgttgt tccttgttgc 120

atctccttt 129

<210> 70
<211> 125
<212> DNA
<213> oligonucleotide probe

<400> 70
ggcgcgcggc ccgcgcgccg gatgagtcct gagtaagatg agtcctgagt aacgctttct 60

gtacgtcccg ggcatagtga attgggaatt ggtacgcagt cgttgttcct tgttgcatct 120

ccttg 125

<210> 71
<211> 122
<212> DNA
<213> oligonucleotide probe

<400> 71
ggcggccgcc gggcgggccg gatgagtcct gagtaagatg agtcctgagt aacggcaaag 60

tatcaggaat gttagcgagg gggaattggt acgcagtctg caacagttga atagaaagat 120

gt 122

<210> 72
<211> 118
<212> DNA
<213> oligonucleotide probe

<400> 72
ggcggccgcc gggcgggccg gatgagtcct gagtaagatg agtcctgagt aacggcaaag 60

tatcaggaat gttagcgagg gaattggtac gcagtccaac agttgaatag aaagatgc 118

## Description

### Field of the invention

The present invention relates to the field of molecular biology and biotechnology. In particular the invention relates to the field of nucleic acid detection, more in particular to the design and composition of (collections) of probes that can be used for the detection of nucleic acids. The invention also relates to methods for the detection of nucleic acids using the probes and compositions. The invention further provides for probes that are capable of hybridising to a target sequence of interest, primers for the amplification of ligated probes, use of these probes and primers in the identification and/or detection of nucleotide sequences that are related to a wide variety of genetic traits and genes and kits of primers and/or probes suitable for use in the method according to the invention. The invention finds applicability in the field of the detection of target nucleotide sequences, whether from artificial, plant, animal or human origin or combinations thereof.

### Background of the invention

There is a rapidly growing interest in the detection of specific nucleic acid sequences. This interest has not only arisen from the recently disclosed draft nucleotide sequence of the human genome and many other genomes and the presence therein, as well as in the genomes of many other organisms, of an abundant amount of single nucleotide polymorphisms (SNP), but also from marker technologies (such as AFLP), SNPWave and the general recognition of the relevance of the detection of specific nucleic acid sequences as an indication of, for instance, genetically inheritable diseases. The detection of the various alleles of the breast cancer gene BRCA 1 to screen for susceptibility for breast cancer is just one of numerous examples. The recognition that the presence of single nucleotide substitutions (and other types of genetic polymorphisms such as small insertion/deletions; indels) in genes provide a wide variety of information has also attributed to this increased interest. It is now generally recognised that these single nucleotide substitutions are one of the main causes of a significant number of monogenically and multigenically inherited diseases, for instance in humans, or are otherwise involved in the development of complex phenotypes such as performance traits in plants and livestock species. Thus, single nucleotide substitutions are in many cases also related to or at least indicative of important traits in humans, plants and animal species.

Analysis of these single nucleotide substitutions and indels will result in a wealth of valuable information, which will have widespread implications on medicine and agriculture in the widest possible terms. It is for instance generally envisaged that these developments will result in patient-specific medication. To analyse these genetic polymorphisms, there is a growing need for adequate, reliable and fast methods that enable the handling of large numbers of samples and large numbers of (predominantly) SNPs in a high throughput fashion, without significantly compromising the quality of the data obtained. One of the principal methods used for the analysis of the nucleic acids of a known sequence is based on annealing two probes to a target sequence and, when the probes are hybridised adjacently to the target sequence, ligating the probes.

The OLA-principle (Oligonucleotide Ligation Assay) has been described, amongst others, in US 4,988,617 (Landegren *et al.).* This publication discloses a method for determining the nucleic acid sequence in a region of a known nucleic acid sequence having a known possible mutation. To detect the mutation, oligonucleotides are selected to anneal to immediately adjacent segments of the sequence to be determined. One of the selected oligonucleotide probes has an end region wherein one of the end region nucleotides is complementary to either the normal or to the mutated nucleotide at the corresponding position in the known nucleic acid sequence. A ligase is provided which covalently connects the two probes when they are correctly base paired and are located immediately adjacent to each other. The presence, absence or amount of the linked probes is an indication of the presence of the known sequence and/or mutation.

Abbot *et al.* in WO 96/15271 developed a method for a multiplex ligation amplification procedure comprising the hybridisation and ligation of adjacent probes. These probes are provided with an additional length segment, the sequence of which, according to Abbot *et al.,* is unimportant. The deliberate introduction of length differences intends to facilitate the discrimination on the basis of fragment length in gel-based techniques.

WO 97/45559 (Barany *et al.)* describes a method for the detection of nucleic acid sequence differences by using combinations of ligase detection reactions (LDR) and polymerase chain reactions (PCR). Methods are disclosed comprising annealing allele-specific probe pairs to a target sequence and subsequent ligation with a thermostable ligase. Amplification of the ligated products with fluorescently labelled primers results in a fluorescently labelled amplified product. Detection of the products is based on separation by size or electrophoretic mobility or on an addressable array.

Other variants of OLA-based techniques have been disclosed *inter alia* in Nilsson *et al.* Human mutation, 2002, 19, 410-415; Science 1994, 265: 2085-2088; US 5,876,924; WO 98/04745; WO 98/04746; US 6,221,603; US 5,521,065; US5,962,223; EP 185494 B1; US 6,027,889; US 4,988,617; EP 246864 B1; US 6,156,178; EP 745140 B1; EP 964704 B1; WO 03/054511; US 2003/0119004; US 2003/190646; EP 1313880; US 2003/0032016; EP 912761; EP 956359; US 2003/108913; EP 1255871; EP 1194770; EP 1252334.

One of the more recently developed modifications in the OLA or LDR technology for the detection of target sequences is related to the use of mobility modifiers (US2003/0119004A1; US 5,470,705). Such mobility modifiers are used in OLA-assays with detection systems based on length, such as electrophoretic devices. Mobility modifiers are polymeric (for instance PEO or PEG) or protein like structures. These modifiers are linked to the probes or to the primers that are used to amplify ligated probes. In an alternative variant, the ligated probe contains a unique and addressable sequence. A detection probe is provided that is capable of hybridizing to the (unique and addressable sequence of the) ligated probe. The detection probe is also provided with a mobility modifier. Subsequently the detection probe is detected.

The mobility modifiers are used to provide a pre-determined mobility to a selected target. Mobility modifiers increase the reliability of the detection step compared to the conventional detection of ligated probes in which the presence of target specific sequences (and the variation therein) introduces a source of variation in mobility that may compromise the successful detection of the presence, absence or amount of the target sequence in the sample.

One of the disadvantages associated with the use of non-nucleotide based mobility modifiers is the limited applicability. Only in combination with detection platforms that are specifically tuned to these mobility modifiers, the assays based on the mobility modifiers can perform with the claimed accuracy. A further disadvantage is that specific mobility modifiers need to be developed and/or validated for a specific platform which hampers the flexibility of the user to select other detection platforms.

The use of nucleotide based mobility modifiers (also known as conventional 'stuffers' or 'identifiers) presents different disadvantages. The current OLA technology is based on the ligation of two non-circularizing linear probes, each containing a target specific part and a primer binding site whereby the two target specific parts are ligated if annealed adjacent on the target sequence. Amplification of the ligated product requires amplification of the complete ligated probe. Also in case of circularizable probes (padlock probes), the entire section of the ligated probe that contains the target specific sequences, the primer sites and the identifier are amplified. As probes have a minimum length needed for adequate annealing to the target sequence and adequate binding of, usually two, amplification primers (usually each about 20 base pairs), ligated probes start at a length of about 80-90 nucleotides. Incorporation of nucleotide based stuffers for discrimination purposes only increases the length of the ligated probes. This not only reduces the throughput capacity on length based platforms, but the co-amplification of fragments of varying length is usually more efficient for shorter fragments than for longer fragments. This introduces a further problem that may become very relevant in case of quantitative analysis of nucleic acid samples.

The use of hybridization probes that uniquely label specific probes (detection probes) requires an additional hybridization step in the detection phase, which is a disadvantage as the additional step introduces extra validation steps that are tedious and lead to increasing costs.

From the discussion of the OLA technology it is clear that there is a need for oligonucleotide probes that combine the advantages and avoid the specific disadvantages of the various ligation probe types described herein. There is also a need for further improvement of the technology by providing probes that have additional advantages. It is one of the goals of the present invention to provide such probes. It is another goal of the present invention to avoid the disadvantages of the commonly known probes as mentioned hereinbefore. It is a further goal of the invention to provide for probes that are suitable for high throughput detection methods. It is also a goal of the present invention to provide for efficient, reliable and/or high throughput method for the detection of target nucleotide sequences, preferably by performing oligonucleotide ligation assays.

The present inventors have set out to eliminate or at least diminish the existing problems in the art while at the same time attempting to maintain the many advantageous aspects thereof, and to further improve the technology. Other problems in the art and solutions provided thereto by the present invention will become clear throughout the description, the figures and the various embodiments and examples.

### Description of the invention

In certain embodiments, methods for determining the presence, absence or amount of at least target nucleic acid sequence in a sample are provided. In certain embodiments, the method comprises providing to the sample a pair of probes for each target nucleic acid sequence to be detected in the sample. In certain embodiments, the pair comprises at least one first probe for each target nucleic acid sequence to be detected in the sample.

In certain embodiments, the pair comprises at least one second probe for each target nucleic acid sequence to be detected in the sample. In certain embodiments, the first probe comprises a first target specific section that is complementary to a first part of the target nucleic acid sequence. In certain embodiments, the second probe comprises a second target specific section that is complementary to a second part of the target nucleic acid sequence. In certain embodiments, the first and second parts of the target nucleic acid sequence are located adjacent to each other. In certain embodiments, the probes in each pair are suitable for ligation together when hybridized adjacent to one another on the target nucleic acid sequence. In certain embodiments, the probes can be ligated when the first and second probe are located adjacent on the target sequence. In certain embodiments, the second probe further comprises a tag section that is essentially non-complementary to the target nucleic acid sequence. In certain embodiments, the tag section comprises a first primer-binding sequence and optionally a second primer-binding sequence.

In certain embodiments, the tag section comprises an identifier sequence. In certain embodiments, the first primer binding sequence is located between the second target-specific section and the identifier. In certain embodiments, the tag section comprises a second primer binding site. In certain embodiments, the identifier is located between the first and second primer binding site.

In certain embodiments, the first and second target specific sections of the first and second probe are allowed to anneal to the respective first and second parts of the target nucleic acid sequence. In certain embodiments,the first and second target specific sections of the probes are annealed adjacent on the target nucleic acid sequence.

In certain embodiments, means are provided for connecting the first and second target specific sections annealed adjacently to the target nucleic acid sequence. In certain embodiments, the first and second target specific sections are allowed to be connected, to produce a connected probe corresponding to the target nucleic acid sequence in the sample.

In certain embodiments, any second probes that have not been connected are removed. In certain embodiments, any non-connected first probes and/or connected probes are isolated/purified/separated.

In certain embodiments, a pair of primers is provided comprising a first primer that is complementary to the first primer-binding sequence and a polymerase enzyme. In certain embodiments, a second primer is provided that is complementary to the second primer-binding sequence. In certain embodiments, the resulting mixture is amplified by elongation of the primer(s) to produce an amplified sample comprising amplicons that are representations of the connected probes.

In certain embodiments, the presence, absence or amount of the at least one target sequence in a sample is determined by detecting the presence, absence o amount of the corresponding amplicon.

The present invention provides for a high throughput method for the detection of the presence, absence or amount of target nucleotide sequences. The method comprises contacting the target sequence with a pair of at least two probes, a first probe that contains a section that is complementary to a first part of the target sequence and a second probe that contains a section that is complementary to a second part of the target sequence. The second probe further contains a tag section either comprising one primer binding site and an optional identifier sequence, wherein the primer binding section is located between the target specific section and the optional identifier sequence or comprising two primer binding sites flanking an optional identifier sequence. When the two probes are annealed to adjacent sections of the target sequence and the respective ends of the probes are located adjacent, they can be ligated. After ligation the unligated second probes are removed and/or the unligated first probes and ligated probes are separated. Amplification with primers capable of hybridizing to the primer binding sites provides relative short sequences that, upon detection on various platforms, indicate the presence of the target sequence of interest.

### Detailed description of the invention

The present invention in a first aspect pertains to a method for determining the presence, absence or amount of at least one target nucleic acid sequence in a sample, wherein the method comprises the steps of:
a) providing to the sample a pair of probes for each target nucleic acid sequence to be detected in the sample, wherein the pair comprises at least one first probe comprising a first target-specific section that is complementary to a first part of the target nucleic acid sequence, and at least one second probe comprising a second target-specific section that is complementary to a second part of the target nucleic acid sequence, wherein the probes in each pair are suitable for ligation together when hybridized adjacent to one another on the target nucleic acid sequence, and whereby the second probe further comprises a tag section that is essentially non-complementary to the target nucleic acid sequence, whereby the tag section comprises a first primer-binding sequence, optionally an identifier sequence and optionally a second primer-binding sequence, wherein the first primer binding sequence is located between the second target-specific section and the identifier;
b) allowing the first and second target-specific sections of the first and second probe to anneal to the respective first and second parts of the target nucleic acid sequence;
c) providing means for connecting the first and second target specific sections annealed adjacently to the target nucleic acid sequence;
d) allowing the first and second target specific sections to be connected, to produce a connected probe corresponding to the target sequence in the sample;
e) removing first and/or second probes that have not been connected;
f) optionally, isolating/purifying/separating any non-connected probes and/or connected probes;
g) providing a first primer that is complementary to the first primer-binding sequence;
h) optionally, providing a second primer that is complementary to the second primer-binding sequence;
i) providing a polymerase enzyme and amplifying the resulting mixture by elongation of the primer(s) to produce an amplified sample comprising amplicons that are representations of the connected probes;
j) determining the presence, absence or amount of the target sequence in the sample by detecting the presence, absence or amount of the corresponding amplicon.

One aspect of the present invention pertains to the advantageous design of the probes used in the present invention. These probes are discussed in more detail herein below.

Conventional (non-circularizable) OLA probes have a structure that can generally be described as symmetric. Symmetric in the sense that a conventional pair of OLA probes commonly follows: "Primer Binding Site 1-Target Specific Part 1" and "Target Specific Part 2- Primer Binding Site 2". After annealing and ligation, the ligated probe has the symmetric structure "Primer Binding Site 1-Target Specific Part 1-Target Specific Part 2- Primer Binding Site 2". Circularizable probes have a structure that can be described as "Target Specific Part 1-Primer Binding Site 1-Primer Binding Site 2 - Target Specific Part 2". Optionally these probes can be further equipped with (nucleotide) stuffers, ZIP sequences, identifiers and the like, which are generally located between one or both of the primer binding sites and the target specific site. See for example WO 97/45559; WO 03/054511 and from the present applicant WO 03/052140 and WO 03/60163.

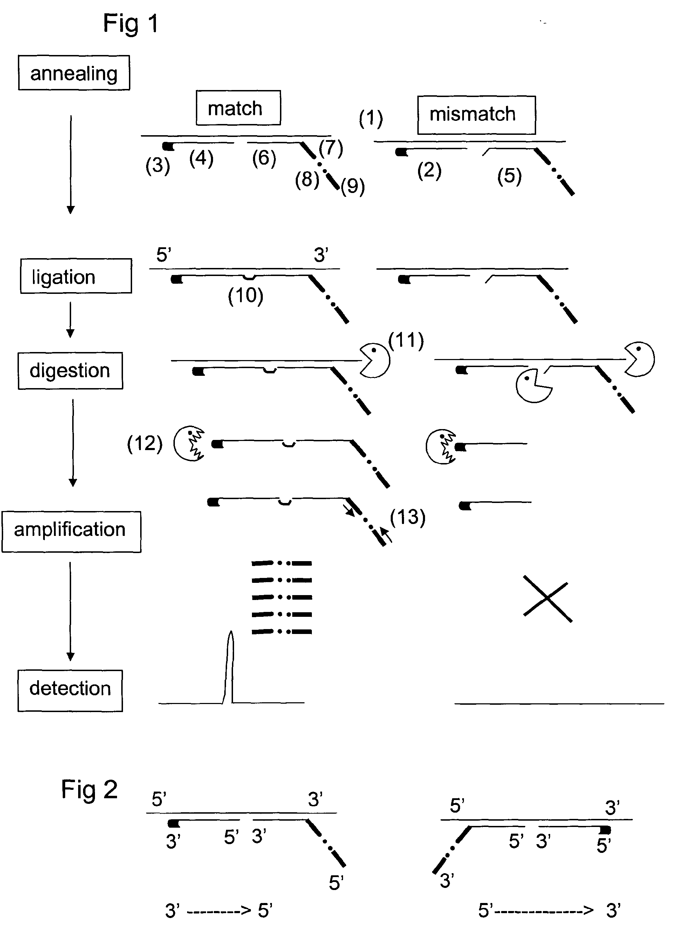

The probes in the pair of the present invention are different from conventional OLA probes (see Fig 1). One probe, generally indicated as the first probe of the present invention comprises a first target specific section. The first probe may also comprise other components as will be discussed below, but preferably the first probe does not comprise a primer binding sequence. The other probe, generally indicated as the second probe, comprises a second target specific section and, generally, two primer binding sites that flank an optional identifier sequence. After annealing and ligation, at least the unligated (second) probes are removed and the remaining connected probes are amplified. Due to the structure of the probes only the section of the second probe is amplified that does not contain the target specific section. This provides for relatively short amplified sequences of known constitution and composition (compared to conventional (non)-circularizable probes) that can be detected. Being shorter, the amplification will be more efficient than with conventional ligated probes. Being of known constitution and composition, detection parameters such as sequence, mass, mobility etc. are known and can be optimised beforehand (i.e. when the assay is designed).

In step a) of the method, a pair of probes comprising at least two probes is provided. A pair of probes is provided to the sample that comprises, or is suspected to comprise at least one target sequence. Each pair preferably comprises two probes, a first probe and a second probe. For certain embodiments such as detection of specific alleles or allele frequencies, multiple second probes may be provided in the pair. The first probe typically comprises and preferably consists of a first target specific section. The first target specific section is complementary (i.e. is capable of hybridising) to a first part of the target nucleic acid sequence. The first probe may contain other sections as disclosed herein elsewhere, but in certain preferred embodiments, the first probe does not contain a primer binding sequence. The second probe typically comprises a second target specific section and a tag section. The second target specific section is complementary (i.e. is capable of hybridising) to a second part of the target nucleic acid sequence. Preferably, the tag section is not complementary to the target sequence and preferably comprises a first primer binding sequence, an identifier and an optional second primer binding sequence. The first primer binding site is preferably located between the identifier and the second target specific section i.e. the structure of the second probe is typically: "second target specific section- first primer binding sequence - identifier - optional second primer binding site". Preferably, the first and second parts on the target sequence are located adjacent, but alternative embodiments are disclosed herein elsewhere in which the first and second parts on the target sequence are separated by one or more nucleotides or in which the probes overlap at the foreseen point of ligation.

In step b) of the method, the probes are brought into hybridizing contact with the target sequence. The probes are allowed to anneal to the target sequence under such conditions that the first and second target specific sections are capable of annealing to the respective first and second parts of the target nucleic acid sequence. Preferably the conditions are such that the first and second target specific sections of the probes directed to one target sequence selectively anneal to that one target sequence and preferably not to any other target sequence in the sample.

In step c) of the method, means are provided for connecting the first and second target specific sections that are annealed adjacently on the target sequence. Such means can be enzymatic or chemical as describe herein elsewhere. The means are capable of connecting (preferably covalently) the respective first and second probes of which the first and second target specific sections are annealed adjacently on the target sequence. Adjacent in this respect means that the probes are annealed such to the target sequence that, apart form any bonding between the first an second probe, the probes are contiguous on the target sequence.

In step d) of the method, the probes that are annealed adjacently to the target sequence are connected using the means for connecting the probes of step c). In case an enzyme is used such as a ligase, this connecting of the probes is generally referred to as ligating, but the term ligation may also be employed when other means are used for connecting the probes.

In step e) of the method, the second probes that have not been ligated or connected to the first probes are removed from the sample or, alternatively or in conjunction therewith, the connected probe is isolated in combination with optional step f), the isolation/purification/separation of any non-connected or unligated first probes.

In certain embodiments, the second probes are removed from the sample by exonuclease treatment of the sample. In certain embodiments, the non-connected second probes are removed by exonuclease treatment of the sample in step (e).The exonuclease treatment removes all unligated second probes. In certain embodiments, the unligated second probes are removed to an extent such that they do not interfere with the detection. To prevent the first probes and the connected probes from being digested by the exonuclease, the first probes have been rendered exonuclease resistant. In certain embodiments, the first probe is rendered exonuclease resistant at a position distal from the end of the first probe that is annealed adjacent to the end of the target specific section of the second probe. The first probe may be rendered exonuclease resistant at any position of the first probe with the exception of the nucleotide at the point of ligation so as not to hinder the ligation step. As the connected probes also contain the first probe, they are also exonuclease resistant.

In optional step f) of the method, the first probes and the connected probes are removed/isolated/purified from the sample. To achieve this, the first probe can be provided with an affinity ligand such as a biotin, optionally through a spacer. The first probes and the connected probes can be isolated from the sample using the strept(avidin)-biotin complex.

In certain embodiments, the removal of unligated second probes and isolation of connected probes and /or unligated first probes can be combined. The biotin/streptavidin combination, and sometimes biotin itself provides resistance to certain exonucleases (FEN1, Murante *et al.* J. Biol. Chem 1995, 270, 30877-30893). Therefore biotinylated probes can be used in the removal of unligated second probes and target sequences by exonuclease. To this end, the biotinylated probe, either before or after being ligated to (strept)avidin, is subjected to the exonuclease treatment that removes all unligated second probes and target sequences but does not digest any biotinylated probe or any biotinylated probe that is (strept)avidin bound. In certain embodiments, the isolation and exonuclease treatment can be carried out simultaneously, depending on the practical circumstances of the two steps such as stringency, buffer composition, temperature etc.

In certain embodiments, the first probe can be attached to a physical carrier such as an array, bead or a paramagnetic particle, preferably via biotin labelling. In certain embodiments, after ligation, the unligated second probes can be removed using known procedures such as washing after which the remaining connected probes and the unligated first probes may be subjected to an amplification step.

In certain embodiments, the first probe can contain a universal nucleotide sequence that is identical for at least a group of first probes. In certain embodiments, the universal nucleotide sequence is located at the end of the probe that is located distal from the point of ligation. The first probes and the connected probes can be isolated by a hybridisation step such as is known as Hybridisation Based Pullout (US601240092).

In certain embodiments, after removal of the non-ligated probes, either by exonuclease treatment of the sample after the ligation step to remove or reduce the non-ligated probes or by isolation of first and/or connected probes by affinity ligands or by hybridization, the sample comprising the connected probes is contacted in step g) with a first primer and optionally in step h) with a second primer. In certain embodiments, step g) and h) can be combined and a pair of primers can be provided comprising a first and a second primer.

In step i) of the method, the resulting mixture comprising connected probes and primer(s) is provided with a polymerase and amplified. Amplification occurs by elongation of the primer(s) to produce an amplified sample. Amplification by elongation of the primer can be linear or exponential, depending on the presence of one or two primer binding sites in the second probe and/or the presence of one or two primers in the amplification step. The primer(s) amplify the section comprising the primer binding site(s) and the (intermediate) identifier sequence to provide amplicons. The identifier sequence identifies the associated connected probe and hence the associated target sequence. The relatively short identifier sections are capable of uniquely identifying the associate target sequence. Due to the decreased length of the identifier sections, these sections amplify efficiently and economically. The resulting amplicons are representations of the connected probes. The amplicons are also indicative of the presence of the target sequence in the sample.

In step j) of the method of the invention, the presence, absence or amount of the target sequence in the sample is determined by detecting the presence, absence or amount of the corresponding amplicon. Detecting the presence, absence or amount of the amplicons in the amplified sample indicates the presence, absence or amount of the target sequence in the nucleic acid sample.

The amplicons have the advantage that they are of a predetermined nucleotide sequence. The predetermined nucleotide sequence of the amplicon (or of the tag section, i.e. the primer sequence(s) and the identifier sequence, of the second probe prior to amplification) and their complements, facilitates the detection and the optimisation thereof as parameters relevant for detection such as length, mass, hybridisation characteristics of the amplicons etc., are known beforehand. This allows for the optimisation of the detection step of the assay, regardless of the target sequence that is to be detected. A large library of suitable sequences can be designed that can serve as amplicons (tag sections). Upon design of an assay, the target sequence to be detected can be compared with the amplicon (tag section) library to avoid unwanted interference of the sequence of the amplicon (tag section) with the target sequence that may lead to compromised detection (false positives or false negatives).

The primer binding sites and the intermediate identifier have been specifically designed to be predictable from one assay to another. In each assay, regardless of which target sequence is to be detected, the amplicons resulting from the ligation-amplification routine can be exactly the same as in another assay directed to the detection of an entirely different target sequence. In practice this means that the sequence of the amplicon is exactly known and has an exactly known mobility, molecular mass, and hybridisation characteristics etc. This allows for the precise and specific detection of the amplicon. This is contrary to the conventional OLA techniques wherein this was not possible due to the presence of sections in the amplified product that were derived from the target sequence. Especially in length based detection this variation causes different mobilities for fragments of otherwise the same (base pair) length, same primer site and same identifier sequence. This variance in mobility is a serious impediment for highly automated, high throughput detection methods that rely on automated scoring software for the analysis of the results. The variance in amplification efficiency is also detrimental in the case of quantitative applications of this technology. The present invention provides also a solution to that end.

### Probe

The sections of the oligonucleotide probes that are complementary to the target sequence are designed such that for each target sequence in a sample, a pair of a first and a second probe is provided, whereby the probes each contain a section at their extreme end that is complementary to a part of the target sequence (a first and a second part of the target sequence, respectively) and the corresponding complementary parts of the target sequence are preferably located essentially adjacent to each other.

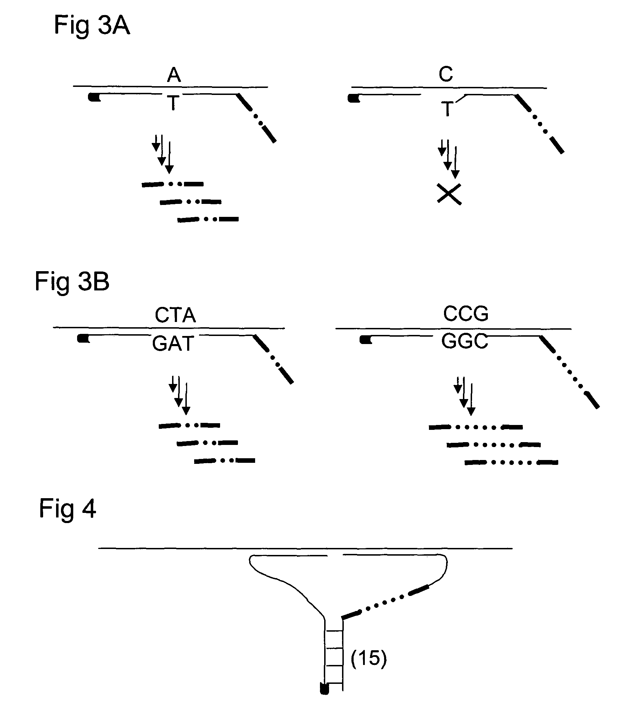

In certain embodiments, additional first and/or second probes can be provided, corresponding to different alleles of a locus. In certain embodiments, the allele specific nucleotide is located at the position of either the first or the second probe at which ligation is to occur, i.e. at the end of the target specific section (see Fig 3A).

In certain embodiments, within a pair of oligonucleotide probes, the first oligonucleotide probe has a section at its 5'-end that is complementary to a first part of a target sequence and the second oligonucleotide probe has a section at its 3'-end that is complementary to a second part of the target sequence. Thus, when the pair of probes is annealed to complementary parts of a target sequence the 5'-end of the first oligonucleotide probe is essentially adjacent to the 3'-end of the second oligonucleotide probe such that the respective ends of the two probes may be ligated to form a phosphodiester bond or covalently connect in an other suitable fashion.

In certain embodiments, within a pair of oligonucleotide probes, the first oligonucleotide probe has a section at its 3'-end that is complementary to a first part of a target sequence and the second oligonucleotide probe has a section at its 5'-end that is complementary to a second part of the target sequence. Thus, when the pair of probes is annealed to complementary parts of a target sequence the 3'-end of the first oligonucleotide probe is essentially adjacent to the 5'-end of the second oligonucleotide probe such that the respective ends of the two probes may be ligated to form a phosphodiester bond or covalently connect in an other suitable fashion. See also Fig 1.

In certain embodiments, for each target sequence for which the presence, absence or amount in a sample is to be determined, a specific pair of first and second oligonucleotide probes is designed, each probe with a section complementary to the adjacent complementary part of each target sequence, as described above. Thus, in the method of the invention, for each target sequence that is present in a sample, a corresponding (specific) amplicon may be obtained in the amplified sample (Fig 3B). In certain embodiments, a multiplicity of first and second oligonucleotide probes complementary to a multiplicity of target sequences in a sample is provided. A pair of first and second oligonucleotide probes for a given target sequence in a sample will at least differ in nucleotide sequence from probe pairs for other target sequences, and will preferably also differ in length and/or mass from probe pairs for other targets, more preferably a probe pair for a given target will produce a connected probe and/or amplicon that differs in length, sequence and/or mass from connected probes corresponding to other targets in the sample as described below. In certain embodiments, amplicons corresponding to different targets may have an identical length or sequence if they can be otherwise distinguished e.g. by different labels as described herein.

### First probe

The first probe comprises a target specific section that is complementary to a first part of the target sequence to be detected in the nucleic acid sample. In certain embodiments, the first probe does not comprise a primer binding sequence.

In certain embodiments, the first probe is exonuclease resistant, preferably at the end distal from the potential ligation site to the second probe. In certain embodiments, the first probe can be made partially exonuclease resistant by introducing the means for exonuclease resistance (such as the herein described thiophosphate linkages between nucleotides) between the nucleotides of the first probe that are not distal to the potential ligation site, for instance somewhere in the middle, as long as the potential ligation site is not compromised. In this embodiment, part of the first probe as well as the part of the connected probe corresponding to said part of the first probe is digested by the exonuclease. However, the ligation site remains intact and this probe design can be equally effective compared to the probe design wherein the exonuclease resistance is provided at the end distal form the ligation point.

The first probe can be rendered exonuclease resistant by any method known in the art. One of the preferred methods is by introducing thiophosphate linkages between nucleotides in the first probe. WO 90/15065, describes a method for making exonuclease-resistant oligonucleotides by incorporating two or more phosphoramidite and phosphoromonothioate and/or phosphorodithioate linkages at the 5' and/or 3' ends of the oligonucleotide. WO 91/06629 describes oligonucleotide compounds with one or more phosphodiester linkages between adjacent nucleotides replaced by a formacetal/ketal type linkage which are capable of binding RNA or DNA. Other methods comprise the blocking of the 3' terminal hydroxyl group by a phosphoryl or acetyl group or as disclosed in U.S. Pat. No. 4,656,127. Shaw et al., 1991, Nucleic Acids Research, 19, 747-750 discloses synthesis of 3' exonuclease-resistant oligonucleotides containing 3' terminal phosphoroamidate modifications). Exonuclease resistance can also be provided by affinity ligand labelling. PNAs (peptide nucleic acids, describes elsewhere herein) and LNAs (locked nucleic acids) are also known to exhibit resistance to exonuclease degradation (Raney *et al.* (1998) in *Peptide Nucleic Acids* (Nielsen, P. E., and Egholm, M., Eds.) Horizon Scientific Press, Wymondham, U.K.; Simeonov *et al.,* Nucl. Acids Res. 2002, vol. 30, e31; Jacobsen *et al.* Int. Biot. Lab, Feb 2001, 18). Other nucleoside alternatives are also suitable in as far as they are capable of providing exonuclease resistance.

In certain embodiments, the first probe is provided with an affinity ligand, preferably at the end distal from the potential ligation site to the second probe. In principle any type of affinity labelling can be used, such as affinity ligands. One suitable combination is (strept)avidin-biotin, wherein the biotin group is coupled to the first probe. Another suitable combination employs antibodies or antigens. In certain embodiments, it is possible to combine the presence of means for exonuclease resistance (such as phosphorothioate linkages) and the affinity labelling in one probe and/or at the same position in the probe.

In certain embodiments, the first probe is provided with an additional nucleotide section preferably at the end distal from the potential ligation site to the second probe. Preferably, the additional nucleotide section is not capable of hybridizing to other parts of the target sequence and/or of the second probe. The unligated first probes and the connected probes can be purified/isolated/removed from the sample by hybridization to the complement of the additional nucleotide section. The complement of the additional nucleotide sequence can be provided on a suitable carrier such as a glass slide, bead or a filter. Examples of such techniques are known and described herein elsewhere as Hybridisation Based Pullout. In certain embodiments, the additional nucleotide section may be useful in a pre-amplification of the ligated probes. The additional nucleotide section can then be used as a primer binding site and together with the second primer binding site, the connected probes can be amplified, preferably prior to any exonuclease treatment. This aids in facilitating selective amplification as disclosed herein in case of high multiplex ligation in combination with lower multiplex detection or of selective amplification of split samples.

### Second probe

The second probe comprises a target specific section that is complementary to a second part of the target sequence. The second probe further comprises a tag section that is preferably essentially non-complementary to the target specific section. Preferably, the tag section is not capable of hybridizing to the target sequence. Preferably, the tag section is not capable of hybridizing to other target sequences in the nucleic acid sample. In certain embodiments, the first and second part of the target sequence are located essentially adjacent, but as can be seen from certain other embodiments, the first and second parts of the target sequence may overlap or are not located adjacent, and these are within the scope of the present invention.

The tag section comprises a first primer binding site and optionally an identifier or identifier sequence. In certain embodiments, the tag section further comprises a second primer binding site. In certain embodiments, wherein only one primer binding site is provided, the primer binding site is located between the target specific section and the identifier. In certain embodiments, wherein two primer binding sites are provided, the identifier sequence is located between the two primer binding sites. The presence of the identifier sequence in the amplicons resulting from the amplification provides the identification of the presence of the target sequence in the sample after the removal of the non-connected probes or isolation of connected probes. In certain embodiments, the identifier provides a length difference between different probes directed to different target sequences in a sample such that the presence of different target sequences may be based on length (or mobility) based detection such as electrophoretic techniques. In certain embodiments, the identifier provides a sequence difference between different probes directed to different target sequences in a sample such that the presence of different target sequences may be based on sequence-based detection such as hybridisation based detection such as (micro)arrays or by using certain identifying probes. In certain embodiments, the identifier provides a mass difference between different probes directed to different target sequences in a sample such that the presence of different target sequences may be based on mass-based detection such as MALDI-TOF.

In certain embodiments, the tag section may comprise recognition sites for restriction endonucleases. The presence of such restriction sites allows to further reduce the size of the amplicon and thus to further increase the throughput capacity of mass-based or length based detection techniques. After amplification, the amplicons can be restricted for instance to remove one or both the primer binding sites. This is especially advantageous when the identifiers are relative large and such a modification may further increase throughput. When detection is based on mass spectrometry, such modification can be advantageous as the resolution of mass spectrometry favours small molecules (oligonucleotides) over larger molecules. An example of the incorporation of such a restriction site can be found in applicants WO 03/60163.

### Circular or padlock probes

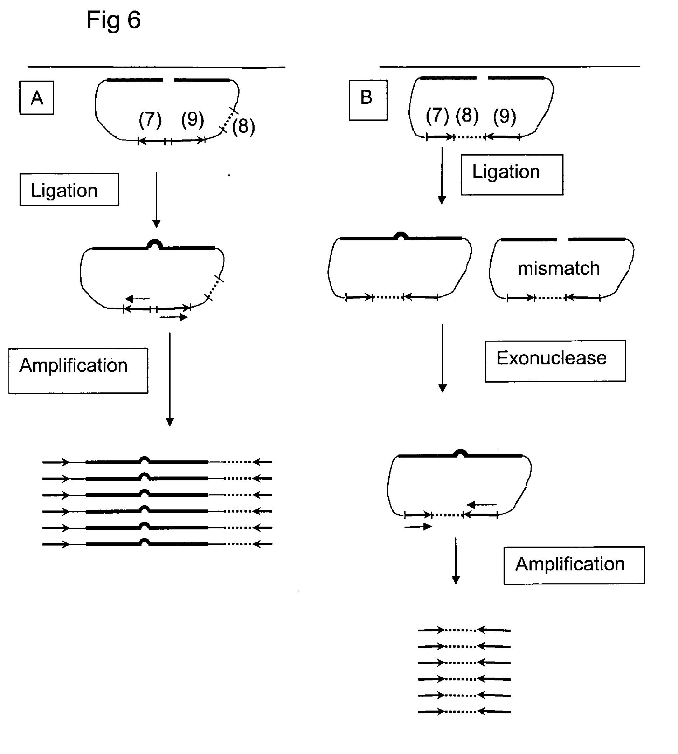

In certain embodiments, of the invention, circular (circularizable) probes or padlock probes can be used. The first and second probes are then combined into one probe. The circular probe is a linear oligonucleotide that, when annealed to the target sequence, and when ligated, has a circular conformation that is topologically locked to the target sequence. In certain embodiments, an exonuclease treatment of the sample after the ligation step and prior to amplification, preferably PCR-amplification, serves to remove any non-ligated circular probes and to prevent any non-ligated probes from amplification. Circularizable probes are themselves known in the art, for instance from EP745140 or from Van Eijk et al, Nucleic Acids Research, 2004, 32, e47. The known probes are commonly amplified using rolling circle amplification resulting in concatamers. Furthermore, the primer binding sites in the known circularizable probes are oriented such that the entire circularised probe is amplified including any target specific sections. In contrast, the primer binding sites in the present circularizable probes are oriented such that preferably only the section comprising the primer binding sites and the optional identifier is amplified and preferably the ligated target specific sections are not amplified. This provides amplicons of relatively short length compared to conventional amplicons obtained from conventionally amplifying circularised probes. This avoids the formation of large concatamers and further unnecessary amplification of the entire circularized probe. See also Fig 6 in this respect.

### Semi-circularizable or Keylock probe

In certain embodiments, for each given target sequence to be detected, preferably at least a pair of two probes is designed such that each probe in the pair is capable of hybridising to a part of the target sequence and the respective probes in the pair further each comprise a section that is complementary to a corresponding section of the other probe in the pair such that both probes are capable of hybridising to each other. The two probes in the pair are designed such that when hybridised to each other they are each also capable of hybridising to a target sequence. When hybridised to each other the two probes mimic or act as padlock probes when used in an oligonucleotide ligation assay for the detection of a target nucleotide sequence, whereas in the subsequent amplification and detection steps the probes function as a linear ligation product. See Fig 4.

In certain embodiments, the first oligonucleotide probe has a section at its 5'-end that is complementary to a first part of a target sequence and the second oligonucleotide probe has a section at its 3'-end that is complementary to a second part of the target sequence. In certain embodiments, the first oligonucleotide probe further comprises a clamp section that is capable of hybridising to a complementary clamp section located in the second oligonucleotide probe whereby the clamp sections are essentially non-complementary to the target sequence. In certain embodiments, the invention pertains to method for determining the presence, absence or amounted at least one target sequence in a sample as outlined herein, where the method comprises the steps of providing a pair of probes comprising clamp sections as outlined herein for each target sequence to be detected. In certain embodiments, the oligonucleotide probes are allowed to anneal to the target sequence, providing means for connecting the first and the second oligonucleotide probes and allowing first and second oligonucleotide probes to be connected to produce a connected probe corresponding to a target sequence in the sample. In certain embodiments,

The clamp section is preferably located at or near the end of the probe that is distal to the target section, i.e. when the target section is located at the 3' end, the clamp section is located more towards the 5' end and vice versa. The clamp section is not necessarily located most distal at the 5'end or 3' end; it may be followed by other sections as discussed herein elsewhere. The clamp sections are preferably designed such that they are not capable of hybridising to the target sections. The clamp sections of the first and second probe of the pair are preferably capable of hybridising to each other. The clamp sections are preferably designed such that two complementary clamp sections have a higher binding affinity for each other than the binding affinity of the target section of the probe for its complementary part in the target nucleotide sequence. This means in practice that the clamp sections, when hybridised to each other, form a stronger duplex than the hybrid between the target section and its complement in the target nucleotide sequence and/or hybridization of complementary clamps takes place at higher temperatures than hybridisation of the target complementary section of the probes to the target. In other words, the hybridised clamp section denatures, under otherwise comparable conditions, at a higher temperature or higher stringency conditions than the denaturation temperature of the target complementary sections in the pair of probes. This allows to choose the conditions during the method of the invention such that the hybridised or locked clamp remains hybridised or closed at least until the probes are connected to produce a connected probe. The locked clamp can be opened by denaturing the (connected) probe at a temperature or under circumstances that allow the denaturation of the locked clamp.

A pair of probes having locked clamps expresses similar or identical hybridisation kinetics and behaviour as do circular or padlock probes. The two probes of a pair can be added separately after which the clamp sections are hybridised to each other in the sample or, alternatively the two probes can be locked prior to being added to the sample.

In a preferred embodiment, the clamp has a denaturation temperature (or melting temperature, Tm) that exceeds the denaturation temperature of the target complementary sections in the pair of probes by at least 1 °C, preferably 5 °C more preferably 10 °C greater than the lowest Tm of the T1 or T2 section. The denaturation temperature of a oligonucleotide sequence can calculated/estimated from the nucleotide composition using the general formula's for Tm = (4*G or C)+(2*A or T) or Tm = (4*G/C)+2*A/T)-5°C (Meinkoth *et al.* Anal. Biochem. (1984) 138: 267-284). Other formulas are likewise applicable as the essence lies in the difference in denaturation temperature between the sections (Tm[clamp]-Tm[target]). This can be achieved not only by varying the length of the clamp sections but also by varying the GC content of the clamp, as a GC basepair increases Tm by about 2 °C compared to an AT basepair. A typical clamp section comprises 10 to 30, preferably 15 to 25 and more preferably 18 to 24 nucleotides. When the GC content is lower, this number of nucleotides may increase as long as the desired hybridisation characteristics are obtained. Alternatively modified nucleotides can be used that increase the hybridisation between the two clamp sections. Examples thereof are nucleotides that have improved hybridisation characteristics, such as Locked Nucleic Acids such as disclosed in WO 99/14226, WO 00/56748, WO 00/66604 and WO 01/25478, Peptide Nucleic Acids or by other molecules that stabilise or enhance DNA hybridisation such as minor groove binders and other, such as those in described in EP 0 974 672.

The GC content of the clamp may vary, wherein the GC content of clamp section ranges from more than 50 to 100%, preferably more than 60%, more preferably more than 70%, most preferably more than 80 % and is preferably in the range of 90-100%. Hence most clamp sections will contain A/T combinations on a more incidental or structural basis. A preferred group of clamp sections are GC enriched ZIP sequences (Iannone *et al.* (2000), Cytometry 39: pp. 131-140). Preferably the clamp section comprises at least one, preferably at least 2, 3, 4, or 5 nucleotides selected from the group consisting of G's and C's, more than each of T1 and T2.

In certain embodiments, when groups of pairs of probes are involved, a different clamp section may be provided for each pair of probes in the group. The clamp section is designed such that a clamp for a first pair of probes and clamps for a second or further pair of probes are distinguishable from each other and preferably do not cross hybridise under conditions used in the ligation assay. Each pair of probes comprises a unique clamp, thereby avoiding cross hybridisation between clamps of different pairs of probes in a sample. To this end the clamp section may comprise additional nucleotides or the oligonucleotide sequences of the clamp section can be unique within the group or a combination thereof, thereby providing for discrimination between two clamps in a group or in order to provide increase the number of possible non-cross binding clamp sequences for use in multiplex ligation assays. This latter embodiment enables the detection of multiple target sequences in one sample simultaneously. This embodiment also enables the detection of one or more different target sequences in multiple samples subsequently using the same collection of pairs of probes. This embodiment enables that the same group of pairs can be used over and over again for the detection of different target sequences.

Preferably, when using different clamps in a group of pairs of probes, these clamps have a Tm that is within a small range, preferably between about 60-90 °C, more preferably between 65-88 °C, most preferably between 70-85 °C. As is known the hybridisation characteristics of nucleic acids are also influenced by the salt concentrations. As used herein, comparison of hybridisation characteristics in general or denaturation temperatures in particular of oligonucleotides is considered under comparable salt concentrations unless indicated otherwise.

Alternative clamps that can be used in the present invention are nucleic acids that contain photodegradable links. After ligation, the photodegradable link is removed and the connected probe amplified and/or detected.

In certain embodiments, the probes of the present invention are equipped with such clamp sections. In certain embodiments, the clamp section of the first and/or the second probe or part thereof is rendered exonuclease resistant as outlined herein elsewhere for other probes. In certain embodiments, the clamp section is provided with affinity ligands as outlined herein elsewhere. In certain embodiments,the clamp section or part thereof can be used for isolation of any unligated first probes and or connected probes based on hybridisation of the clamp section such as disclosed herein elsewhere for hybridisation based pullout or other hybridisation based isolation methods using glass slides, arrays, beads, paramagnetic particles etc. In certain embodiments, the clamp section is denatured prior to exonuclease treatment and/or isolation of unligated first probe or connected probes.

### Identifiers

In certain embodiments, the second oligonucleotide probe of the present invention further comprises an identifier or an identifier sequence. The identifier sequence is an oligonucleotide sequence of a variable length, sequence or mass. The length of the identifier varies from 0 to 1000, preferably from 0 to 500, more preferably from 1 to 100 and most preferred from 1 to 50 nucleotides. The identifier may be a unique sequence as is known as a ZIP-coded sequence as described by Iannone *et al.* (2000), Cytometry 39: pp. 131-140. The identifier is located such that the first primer binding site is located between the second target specific section and the identifier. In case of two primer binding sites, the identifier is located between the two primer binding sites. In case the identifier has length 0, the amplicon to be detected has a length equal to the sum of the two primer lengths. The identifier may be used to impart length differences between probes or connected probes but can also be used to impart mass differences for mass-based detection or addressable sequences (ZIPs and cZIPs) for hybridisation based detection.

### Primer Binding sites

Primer binding sites may be incorporated in the second probe to facilitate amplification, whether linear or exponential. Primer binding sites are preferably located in other parts of the second probe than in the target specific section, preferably in the tag section which is essentially non-complementary to the target sequence. Primer binding sites are capable of binding primers to initiate primer elongation or amplification. Preferably within a group of pairs of probes, the primer binding sites are universal, i.e. only a predetermined group of primer binding sites are incorporated in the probe to enable multiplex primer elongation or amplification from a limited number of primers, such as primers comprising one or more selective bases at their 3' end, such as are known from AFLP (EP 0 534 858). Between groups of pairs of probes, primer binding sites may be different. In certain embodiments, the Tm of primers capable of binding to the different primer binding sites may be different between groups of pairs of probes.

The function of identifier and primer binding sites in a probe can be combined and can be interrelated in the sense that a specific part of the probe may function as (part of) a primer binding site for (selective) primer elongation/amplification, and at the same or another time as (part of) an identifier to impart the desired and detection platform-based difference such as disclosed herein elsewhere.

### Hybridisation

In certain embodiments, the probes are brought into hybridizing contact with the target sequence in the sample. The pairs of oligonucleotide probes are subsequently allowed to anneal to the, preferably adjacent, complementary parts of the target sequence in the sample. Methods and conditions for specific annealing of oligonucleotide probes to complementary target sequences are well known in the art (see e.g. in Sambrook and Russel (2001) "Molecular Cloning: A Laboratory Manual (3^{rd} edition), Cold Spring Harbor Laboratory, Cold Spring Harbor Laboratory Press).

Usually, after mixing of the oligonucleotide probes and target sequences the nucleic acids are denatured by incubation (generally at between 94 °C and 96 °C) for a short period of time (e.g. 30 seconds to 5 minutes) in a salt buffer. The sample containing the denatured probes and target sequences is then allowed to cool to an optimal hybridisation temperature for specific annealing of the probes and target sequences, which usually is about 5°C below the melting temperature of the hybrid between the complementary section (target section) of the probe and its complementary sequence (in the target sequence). In order to prevent aspecific or inefficient hybridisation of one of the two probes of a pair, or in a sample with multiple target sequences, it is preferred that, within one sample, the sections of the probes that are complementary to the target sequences are of a similar, preferably identical melting temperatures between the different target sequences present in the sample. Thus, the complementary sections of the first and second probes preferably differ less than 20, 15, 10, 5, or 2 °C in melting temperature. This is facilitated by using complementary sections of the first and second probes with a similar length and/or similar G/C content, the complementary sections preferably differ less than 20, 15, 10, 5, or 2 nucleotides in length and their G/C contents differ by less than 30, 20, 15, 10, or 5 %. Complementary as used herein means that a first nucleotide sequence is capable of specifically hybridising to second nucleotide sequence under normal stringency conditions. A nucleotide sequence that is considered complementary to another nucleotide sequence may contain a minor amount, i.e. preferably less than 20, 15, 10, 5 or 2%, of mismatches. Alternatively, it may be necessary to compensate for mismatches e.g. by incorporation of so-called universal nucleotides, such as for instance described in EP-A 974 672, incorporated herein by reference or by incorporation of certain modified nucleotides that are capable of compensating for mismatches for instance by increasing the melting temperature or increasing specificity such as LNAs. Since annealing of probes to target sequences is concentration dependent, annealing is preferably performed in a small volume, i.e. less than 25 µl, preferably less than 10 µl. Under these hybridisation conditions, annealing of probes to target sequences usually is fast and does not to proceed for more than 5, 10 or 15 minutes, although longer annealing time may be used as long as the hybridisation temperature is maintained to avoid aspecific annealing. Longer annealing times are more important/required for quantitative applications which rely on complete target occupation by ligation probes in order to allow monitoring or relative amounts of target sequences between samples.

In certain embodiments,of the invention, excellent results have been obtained by prolonged hybridisation times such as overnight hybridisation or by repeated hybridisation, such as 10 cycles of 1 hour. Prolonged hybridisation times can be advantageous in these assays as the difference in signal due to different hybridisation efficiencies is reduced and it is considered desirable to achieve complete hybridisation and ligation of all probes for which a target sequence is present. Excellent results have been obtained by a combined hybridisation-ligation step using a thermostable ligase described herein. In this embodiment the hybridisation-ligation was performed by allowing the probes to hybridise during 1 hour in the presence of a thermostable ligase, followed by a denaturation step. Repeating these steps for at least 2 times provided good results. Repeating these steps 10 times provided excellent results.

To avoid evaporation during denaturation and annealing, the walls and lids of the reaction chambers (i.e. tubes or microtitre wells) may also be heated to at least the same temperature as the reaction mixture which is commonly achieved by the use of commercial DNA amplification equipment or by providing a mineral oil overlay. In preferred oligonucleotide probes the length of the target-complementary section is preferably at least 15, 18 or 20 nucleotides and preferably not more than 30, 40, or 50 nucleotides and the probes preferably have a melting temperature from the target section of at least 50°C, 55°C or 60°C.

### Ligation

The respective 5'-phosphorylated and 3'-hydroxylated ends of a pair of first and second oligonucleotide probes that are annealed essentially adjacent to the complementary parts of a target sequence are connected in step (c) to form a covalent bond by any suitable means known in the art. The ends of the probes may be enzymatically connected into a phosphodiester bond by a ligase, preferably a DNA ligase. DNA ligases are enzymes capable of catalysing the formation of a phosphodiester bond between (the ends of) two polynucleotide strands bound at adjacent sites on a complementary strand. DNA ligases usually require ATP (EC 6.5.1.1) or NAD (EC 6.5.1.2) as a cofactor to seal nicks in double stranded DNA. Suitable DNA ligase for use in the present invention are T4 DNA ligase, *E. coli* DNA ligase or preferably a thermostable ligase like e.g. *Thermus aquaticus* (Taq) ligase, *Thermus thermophilus* DNA ligase, or *Pyrococcus* DNA ligase.

Alternatively, chemical ligation of suitably modified polynucleotide ends may be used to ligate two oligonucleotide probes annealed at adjacent sites on the complementary parts of a target sequence. Exemplary reactive groups on modified polynucleotide ends include, but are not limited to, phosphorothioate and tosylate or iodide, esters and hydrazide, RC(O)S, haloalkyl, RCH2S and [alpha]-haloacyl, thiophosphoryl and bromoacetamide groups, and S-pivaloyloxymethyl-4-thiothymidine.

Chemical ligation agents include, without limitation, activating, condensing, and reducing agents, such as carbodiimide, cyanogen bromide (BrCN), N-cyanoimidazole, imidazole, 1-methylimidazole/carbodiimide/cystamine, dithiothreitol (DTT) and ultraviolet light. Autoligation, i.e., spontaneous ligation in the absence of a ligating agent, is also within the scope of the invention. Detailed protocols for chemical ligation methods and descriptions of appropriate reactive groups can be found, among other places, in Xu et al., Nucleic Acid Res., 27:875-81 (1999); Gryaznov and Letsinger, Nucleic Acid Res. 21:1403-08 (1993); Gryaznov et al., Nucleic Acid Res. 22:2366-69 (1994); Kanaya and Yanagawa, Biochemistry 25:7423-30 (1986); Luebke and Dervan, Nucleic Acids Res. 20:3005-09 (1992); Sievers and von Kiedrowski, Nature 369:221-24 (1994); Liu and Taylor, Nucleic Acids Res. 26:3300-04 (1999); Wang and Kool, Nucleic Acids Res. 22:2326-33 (1994); Purmal et al., Nucleic Acids Res. 20:3713-19 (1992); Ashley and Kushlan, Biochemistry 30:2927-33 (1991); Chu and Orgel, Nucleic Acids Res. 16:3671-91 (1988); Sokolova et al., FEBS Letters 232:153-55 (1988); Naylor and Gilham, Biochemistry 5:2722-28 (1966); and U.S. Pat. No. 5,476,930.

Both chemical and enzymatic ligation occur much more efficient on perfectly matched probe-target sequence complexes compared to complexes in which one or both of the probes form a mismatch with the target sequence at, or close to the ligation site (Wu and Wallace, 1989, Gene 76: 245-254; Xu and Kool, *supra*)*.* In order to increase the ligation specificity, i.e. the relative ligation efficiencies of perfectly matched oligonucleotides compared to mismatched oligonucleotides, the ligation is preferably performed at elevated temperatures. Thus, in certain embodiments,of the invention, a DNA ligase is employed that remains active at 50 - 65°C for prolonged times, but which is easily inactivated at higher temperatures, e.g. used in the denaturation step during a PCR, usually 90 - 100°C. One such DNA ligase is a NAD requiring DNA ligase from a Gram-positive bacterium (strain MRCH 065) as known from WO 01/61033. This ligase is referred to as "Ligase 65" and is commercially available from MRC Holland, Amsterdam. In certain embodiments, a Taq Ligase is used. In certain embodiments, the ligase is inactivated after ligating the first and second probes. In certain embodiments,the connected probe is denatured from the target sequence.

### Gap Ligation

In an alternative embodiment, for instance directed to the identification of indels, the respective ends of the target complementary sections of the first and second probe may be annealed such that a gap is left. In certain embodiments, the first and second parts of the target nucleotide sequence are not located adjacent. In other words, the first and second target specific sections of the first and second probe are not hybridized to first and second parts of the target nucleotide sequence that are located adjacent. This is fundamentally different from other varieties of this technology such as disclosed inter alia in EP 185494, US5521065, US5692223 and WO 03054311. This gap can be filled with a suitable (third) (oligo)nucleotide and ligated. Such methods are known in the art as 'gap ligation' and are disclosed inter alia in WO 00/77260; US5185243; EP439182; EP320308; WO90/01069. Another possibility to fill this gap is by extension of one end of the probe using a polymerase and a ligase in combination with single (labelled) nucleotides, optionally preselected from A, T, C, or G, or di-, tri-or other small oligonucleotides. In case the target sequence is RNA, yet another possibility to fill the gap is by extension of one end of the probe using reverse transcriptase and a ligase in combination with single nucleotides, optionally preselected from A, T, C, or G, or di-, tri- or other small oligonucleotides.

Gap ligation may find application in the detection of multiple SNPs (haplotyping) that are closely located.

### Cleavase ligation

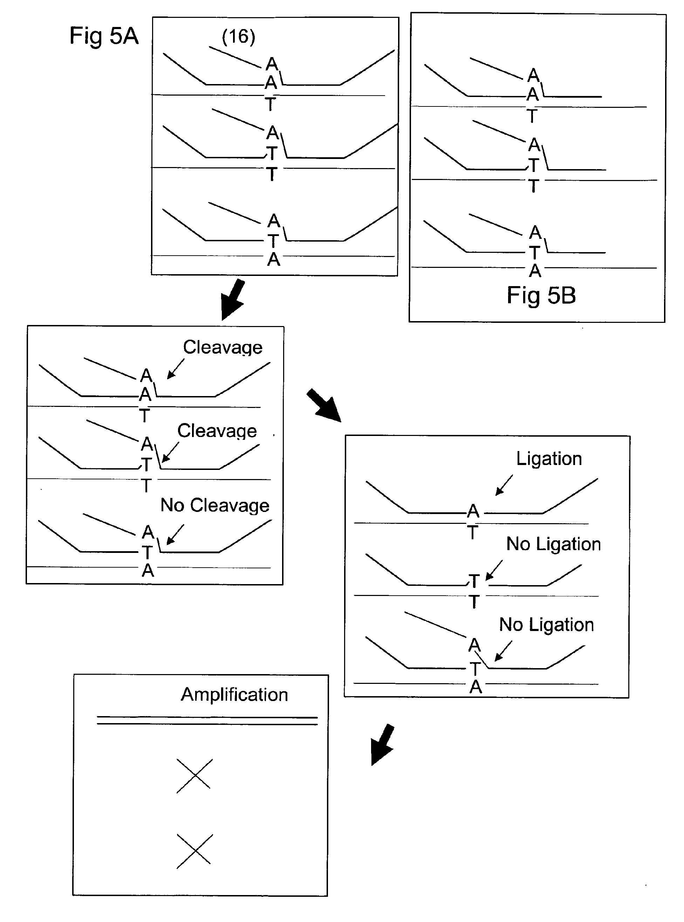

In one aspect of the present invention, an additional discriminating step can be introduced prior to ligation. In certain embodiments, the first or the second probe is designed such that one of the two probes is extended beyond the foreseen point of ligation of its target-specific section. Preferably the probe is extended with a sequence that is not complementary to the target sequence. In the event of correct annealing of target-specific sections of the two probes to the target sequence, a forked cleavage structure is formed wherein the 3'-end of the target-specific section of the non-extended probe is annealed to the target sequence, while the extended 5' end of the other probe, which is non-complementary to the target sequence, forms a single-stranded arm (see Fig. 5). The thus-obtained forked cleavage structure is a substrate for the 5' nuclease activity of DNA polymerases, referred to herein as a cleaving agent, or cleavase. A preferred cleavase is a modified DNA polymerase having 5' nuclease activity but lacking synthetic activity. An example of such a forked cleavage structure and such a cleavase is described in EP 601834 and US 5795763 (Third Wave Technologies).

In certain embodiments the cleavase may be a native DNA polymerase but preferably the cleavase is a modified form that lacks the synthetic activity of the DNA polymerase. Suitable DNA polymerases with 5' nuclease activity and that may be modified to inactivate their synthetic activity are polymerases from e.g. Thermus thermophilus, Thermus aquaticus, Escherichia coli, and Thermus flavus, or a modified form of the gene 6 product from bacteriophage T7. Other suitable cleavases are mentioned *inter alia* in US6635463, US6562611, US6555357, US6458535, US6348314, US6090606, US 6090543, US6001567, US5994069, US5985557, US5843669,US5846717, US5837450, US5614402, WO94/29482, WO97/27214, WO98/23774, WO98/42873.

Upon incubation of the forked cleavage structure with a suitable cleavase, cleavage will occur in the extended probe, right between the first unmatched nucleotide of the extension sequence and the first matched nucleotide of the target-specific section of the extended probe. The extension sequence is thus removed and the two ends of the target-specific sections of the first and second probes will immediately adjacent to each other, in case of a perfect match with the target sequence, thus allowing for ligation of the two probes to form a connected probe (see Fig 5A). This principle is valid for and can be applied to any conventional OLA assay and the assays of the present invention alike (see Fig 5A and 5B) and may form an inventive improvement of the OLA-technology by further improving the fidelity of the OLA-technology. The principle is valid for non-circularizable, circularizable and semi-circularizable probes alike.

In certain embodiments, the general method for the OLA-assays comprises a step wherein a cleavage structure is formed comprising the target nucleic acid sequence, a first probe and a second probe. In certain embodiments, the first probe comprises a first target specific region that is capable of annealing to a first section of the target nucleic acid sequence to form a first duplex. In certain embodiments, the second probe comprises a second target specific region that is capable of annealing to a second section of the target nucleic acid sequence to form a second duplex. In certain embodiments, the first and second sections of the target nucleic acid sequence are contiguous so that the first and the second duplexes are contiguous. In certain embodiments, the first probe or the second probe comprises a further region (16), an extended region, preferably an extended 5'-end, that is not capable of annealing to the target nucleic acid sequence. In certain embodiments, the further (extended) region is located at the end of the first or second probe at the position of the junction site (i.e. the potential site of ligation of the OLA-assay) between the first and second sections of the target nucleic acid sequence. In certain embodiments, the further (extended) region provides a non annealed section of the first or the second probe to thereby create a (forked) cleavage structure. In certain embodiments, exposing the cleavage structure to a cleavage agent that preferably cleaves the cleavage structure in a manner independent of the sequence of the cleavage structure results in cleavage of the cleavage structure when the cleavage structure and cleavage agent are incubated under conditions wherein cleavage can occur. In certain embodiments, cleaving the cleavage structure results in removal of the further (extended) region. In certain embodiments, the removal of the further(extended) region by cleaving the cleavage structure results in adjacent localization of the first and second probe.

In one aspect, the invention relates to the use of a cleavage agent, preferably prior to ligation, in OLA-assays. In certain embodiments, the cleavage agent is used to remove an overhang (i.e. the further or extended region) of the first or second probe located at the envisaged point of ligation such that the first and second probe can be ligated. The characteristics of the cleaving agent are that cleavage occurs when the two probes are annealed adjacent to each other on the target sequence and one of the probes has an overhang at the point where the probes are annealed adjacent. In certain embodiments, cleavage occurs preferably only when the two probes are annealed adjacent to each other on the target sequence and one of the probes has an overhang at the point where the probes are annealed adjacent. The cleavage of the overhang provides two probes that are annealed adjacent on the target sequence and that can be ligated. One of the technical advantages of this cleavage step is that the cleavage step provides the 5' phosphate at the end of one of the probes necessary for ligation. The provision of the 5'phosphate can be used as an alternative for conventional oligonucleotide synthesis wherein phosphorylation at the 5' end is one of the final steps in the synthesis of oligonucleotides. A further technical advantage is that the selectivity and specificity of the subsequent ligation reaction is significantly increased due to the improved selectivity of the cleavage agent to cleave only cleavage structures, i.e. those structures where the nucleotide in the overhang is complementary or capable of hybridizing to the nucleotide in the target sequence.

The introduction of the cleavage step in the OLA assay combines the specificity of the monoplex Invader Assay (Third Wave Technologies) with the flexible multiplex capacity of OLA SNPWave assays. This allows for instance to measure SNP frequencies in pooled or complex samples or other forms of quantitative measurement of sequences such as non-routine transcript profiling, or quantitative measurement of contamination levels of pathogens in soil, food, waters etc.

The use of this additional step in OLA assays provides significant advantages and finds application in, for instance, in the field of quantitative analysis of allele frequencies in, for instance, population screenings or in the field of identification of low-frequent mutants in complex samples.

### Removal of non-ligated second probes and/or isolation of first probes and/or connected probes.

In step e) of the method any second probes that have not been connected are removed. In certain embodiments, the unligated second probes are removed. In certain embodiments, the invention comprises a step for the removal of oligonucleotide probes that are not annealed to target sequences and/or that are not connected or ligated. In certain embodiments, steps are included for the removal of the target sequences, preferably after the ligation step. In certain embodiments, removal of the unligated second probes is carried out prior to primer elongation and/or amplification.

Removal/elimination of the oligonucleotide probes that are not connected/ligated prevents that the unligated second probes contribute to the signal due to primer elongation/amplification that is not related to the occurrence of a ligation event and hence not to the presence of a target sequence in the sample. In certain embodiments, the removal of unligated probes is complete in that 100 % of the unligated second probes is removed or eliminated. In certain embodiments, the removal/elimination of the unligated second probes is such that less than 1 %, preferably less than 0.01 %, more preferably less than 0.001 %, most preferably less than 0.0001% of the original number of second probes for a specific target nucleotide sequences remains. In certain embodiments, the removal is complete in the sense that the remaining unligated second probes do not interfere with the amplification and/or preferably do not lead to adverse results in the sense of false-positive detections. In certain embodiments, the removal is such that not more than 10 % of a normalised detection signal of an amplicon is related to incomplete removal of unligated second probes. In other words, compared to a correct, positive determination of the presence of a target sequence in a sample by detection of the detection signal associated with the corresponding amplicon, the signal associated with a false positive detection is not more than 10 % of the signal of the correct positive detection. In preferred embodiments, the signal is not more than 1%, preferably not more than 0.1% more preferably not more than 0.01 %. Particularly preferred embodiments have a signal that is less than 0.001%, preferably less than 0.0001%, more preferably less than 0.00001 %. In certain embodiments, the signal derived from incomplete removal of the unligated second probes is statistically insignificant compared to the signal derived from the ligated second probes.

One embodiment to eliminate one or more of the non-connected/ligated components without removing the information content of the connected probes utilizes one or more exonucleases to digest non-connected/ligated oligonucleotide probes. In certain embodiments, the target sequences are also removed by the use of exonucleases, which aids in a further increase of the signal to noise ratio. By blocking the end that is not ligated, for example the 3' end of the downstream oligonucleotide probe (the first probe that does not contain a primer binding site), one probe can be made substantially resistant to digestion, while the other is sensitive. Only the presence of full length ligation product sequence will then prevent digestion of the connected probe. The unligated second probe and, in certain embodiments, the target sequence are both digested by the exonuclease. In certain embodiments, the exonuclease is inactivated after removing the unligated second probes.

Blocking groups include use of one or more thiophosphate groups and/or use of 2-O-methyl ribose sugar groups in the backbone. Other blocking groups are disclosed herein before.

In certain embodiments, the exonuclease has a 3'-5' activity. In certain embodiments, the exonuclease has a 5'-3' activity. In certain embodiments, the exonuclease has both a 3'-5' and a 5'-3' activity. In certain embodiments,the exonuclease is capable of digesting ssDNA and/or *ds*DNA, preferably ssDNA. In certain embodiments, the exonuclease is selected from the group of Exonuclease I, Exonuclease III, Exonuclease V, Exonuclease IV, Exonuclease T, Lambda exonucleases, T7 Exonuclease, strandase exonuclease , 3'-5' Exophosphodiesterases. Exonucleases include Exo I (3'-5'), Exo III (3'-5'), and Exo IV (both 5'-3' and 3'-5'), the latter requiring blocking on both sides. Preferably the exonuclease is a 5'-3' exonuclease, more preferably Exo I or Exo III. In the case of an exonuclease that has both a 3'-5' and a 5'-3' activity this can be achieved by also blocking the second probe in a manner similar to the first probe: at a position distal, preferably most distal from the target specific section. In certain embodiments, the second probe can have the following structure: "target-specific section -- first primer-binding site -- identifier --second primer-binding site - blocking group". The blocking group can also be located in the second primer binding site or the identifier (in case of single primer (linear amplification)) with the proviso that position of the blocking group does not interfere with the amplification. This can be achieved with a second primer binding site (identifier) that contains for instance one or more thiophosphate linkages between the nucleotides making up the primer binding site (or the identifier). The bi-directional exonuclease will digest any oligonucleotide that is not protected by two blocking groups, i.e. all unligated first and second probes and the target sequence. Only connected probes remain that can be amplified and detected. Exonuclease treatment of samples of connected probes has been described before in relation to increasing signal to noise ratio by removing non-ligated probes and target sequences. The exonuclease treatment has not yet been disclosed in relation to the discrimination step of the OLA assay.

An alternative embodiment for the separation of ligated from unligated probes is by Hybridization-based pullout (HBP). HBP comprises a process wherein a nucleotide sequence complementary to at least a portion of one probe, for example, the primer-specific portion, is bound or immobilized to a solid or particulate pullout support (see, e.g., U.S. patent 6124092). In the present invention, it is preferably the first probe that comprises a pullout section at the end distal from the envisaged point of ligation. In certain embodiments,the pullout section and the clamp section and/or the primer binding section may be the same or overlap, performing different functions at different moments in the method.

The ligation reaction mixture (comprising the ligation product, target sequences, and unligated probes) is exposed to the pullout support. The ligation product, under appropriate conditions, hybridizes with the support-bound sequences. The unbound components of the ligation reaction mixture are removed, purifying the ligation products from those ligation reaction mixture components that do not contain sequences complementary to the sequence on the pullout support. One subsequently removes the purified ligation products from the support and combines it with at least one primer pair to form a first amplification reaction mixture. The skilled artisan will appreciate that additional cycles of HBP using different complementary sequences on the pullout support will remove all or substantially all of the unligated probes, further purifying the ligation product.

Another alternative embodiment for the separation of the connected probes from the unligated probes is an embodiment wherein one of the probes, preferably the first probe, contains an affinity label and preferably is biotinylated, i.e. contains a biotin. The biotin can be located at the end distal from the point of ligation or can be located internally in the probe at a position that does not interfere with the ligation, i.e. between the nucleotides at both ends. After ligation, the remaining first probes and the ligated probes are isolated from the sample using strept(avidin) or a similar affinity ligand /binding complex combination. The unligated (second) probes remain in the sample. The isolated probes can be subjected to the subsequent steps of the method *inter alia* primer annealing, amplification and detection.

### Target sequences

In its widest definition, the target sequence may be any nucleotide sequence of interest. The target sequence can be any sequence of which its determination/detection is desired, for instance because it is indicative, associated or representative of a certain ailment or genetic make up or disorder. The target sequence preferably is a nucleotide sequence that contains, represents or is associated with a polymorphism. The term polymorphism herein refers to the occurrence of two or more genetically determined alternative sequences or alleles in a population. A polymorphic marker or site is the locus at which sequence divergence occurs. Preferred markers have at least two alleles, each occurring at frequency of greater than 1%, and more preferably greater than 10% or 20% of a selected population. A polymorphic locus may be as small as one base pair. Polymorphic markers include restriction fragment length polymorphisms, variable number of tandem repeats (VNTR's), hypervariable regions, minisatellites, dinucleotide repeats, trinucleotide repeats, tetranucleotide repeats, simple sequence repeats, and insertion elements such as Alu. The first identified allelic form is arbitrarily designated as the reference form and other allelic forms are designated as alternative or variant alleles. The allelic form occurring most frequently in a selected population is sometimes referred to as the wild type form. Diploid organisms may be homozygous or heterozygous for allelic forms. A diallelic polymorphism has two forms. A triallelic polymorphism has three forms. A single nucleotide polymorphism occurs at a polymorphic site occupied by a single nucleotide, which is the site of variation between allelic sequences. The site is usually preceded by and followed by highly conserved sequences of the allele (e.g., sequences that vary in less than 1/100 or 1/1000 members of the populations). A single nucleotide polymorphism usually arises due to substitution of one nucleotide for another at the polymorphic site. Single nucleotide polymorphisms can also arise from a deletion of a nucleotide or an insertion of a nucleotide relative to a reference allele. Other polymorphisms include (small) deletions or insertions of several nucleotides, referred to as indels. A preferred target sequence is a target sequence that is associated with an AFLP® marker, i.e. a polymorphism that is detectable with AFLP®.

### DNA

In the nucleic acid sample, the nucleic acids comprising the target may be any nucleic acid of interest. Even though the nucleic acids in the sample will usually be in the form of DNA, the nucleotide sequence information contained in the sample may be from any source of nucleic acids, including e.g. RNA, polyA⁺ RNA, cDNA, genomic DNA, organellar DNA such as mitochondrial or chloroplast DNA, synthetic nucleic acids, DNA libraries, clone banks or any selection or combinations thereof. The DNA in the nucleic acid sample may be double stranded, single stranded, and double stranded DNA denatured into single stranded DNA. Denaturation of double stranded sequences yields two single stranded fragments one or both of which can be analysed by probes specific for the respective strands. Preferred nucleic acid samples comprise target sequences on cDNA, genomic DNA, restriction fragments, adapter-ligated restriction fragments, amplified adapter-ligated restriction fragments. AFLP fragments or fragments obtained in an AFLP-template preamplification.

### Samples

It is preferred that a sample contains two or more different target sequences, i.e. two or more refers to the identity rather than the quantity of the target sequences in the sample. In particular, the sample comprises at least two different target sequences, in particular at least 10, preferably at least 25, more preferably at least 50, more in particular at least 100, preferably at least 250, more preferably at least 500 and most preferably at least 1000 additional target sequences. In practice, the number of target sequences in a sample that can be analysed is limited, among others, by the number of amplicons than can be detected. E.g., too many different pairs of first and second oligonucleotide probes in a sample may corrupt the reliability of a multiplex amplification step.

A further limitation is formed e.g. by the number of fragments in a sample that can be resolved by the detection platform used. The number can also be limited by the genome size of the organism or the transcriptome complexity of a particular cell type from which the DNA or cDNA sample, respectively, is derived.

### Primers

The connected probe is amplified using a pair of primers corresponding to the primer-binding sites. In certain embodiments, the pair of primers contains only one primer and the amplification is linear rather than exponential. In certain embodiments, the pair comprises a first primer that is capable annealing to the first primer-binding section and capable of initiating amplification or elongation. In certain embodiments, the pair further comprises a second primer that is capable annealing to the second primer-binding section and capable of initiating amplification or elongation. In certain embodiments, the second primer has the same sequence as the second primer binding site in the probe. In a preferred embodiment, at least one of the primers or the same pair of primers is used for the amplification of two or more different connected probes in a sample, preferably for the amplification of all connected probes in a sample. Such a primer is sometimes referred to as a universal primer as these primers are capable of priming the amplification of all connected probes containing the corresponding universal primer binding site and consequently of all ligated probes containing the universal primer binding site. The different primers that are used in the amplification in step (i) are preferably essentially equal in annealing and priming efficiency. Thus, the primers in a sample preferably differ less than 20, 15, 10, 5, or 2 °C in melting temperature. This can be achieved as outlined herein for the target-specific sections of the oligonucleotide probes. Unlike the sequence of the target-specific sections, the sequence of the primers is not dictated by the target sequence. Primer sequences may therefore conveniently be designed by assembling the sequence from tetramers of nucleotides wherein each tetramer contains one A,T,C and G or by other ways that ensure that the G/C content and melting temperature of the primers are identical or very similar. The length of the primers (and corresponding primer-binding sites in the tag section of the second probe) is preferably at least 12, 15 or 17 nucleotides and preferably not more than 25, 30, 40 nucleotides.

In a certain embodiment, at least two of the second oligonucleotide probes that are complementary to at least two different target sequences in a sample each comprise a tag section that comprises a primer-binding section that is complementary to a single primer sequence.

Thus, preferably at least one of the first and second primer in a primer pair is used for the amplification of connected probes corresponding to at least two different target sequences in a sample, more preferably for the amplification of connected probes corresponding to all target sequences in a sample. Preferably only a single first primer is used and in some embodiments only a single first and a single second primer is used for amplification of all connected probes. Using common primers for amplification of multiple different fragments usually is advantageous for the efficiency of the amplification step.

The connected probes obtained from the ligation of the adjacently annealed probe sections are amplified in step (i), using a primer pair, preferably consisting of a pair of primers for each of the connected probes in the sample. The primer pair comprises primers that are complementary to primer-binding sequences that are present in the connected probe. A primer pair usually comprises a first and at least a second primer, but may consist of only a single primer that primes in both directions. Excellent results have been obtained using primers that are known in the art as AFLP -primers such as described inter alia in EP534858 and in Vos *et al.,* Nucleic Acid Research, 1995, vol. 23, 4407-44014 and discussed in more detail herein below.

### Selective primers

In certain embodiments, one or more of the primers used in the amplification step of the present invention is a selective primer. A selective primer is defined herein as a primer that, in addition to its universal sequence which is complementary to a primer binding site in the probe, contains a region that comprises so-called "selective nucleotides". The region containing the selective nucleotides is located at the 3'-end of the universal primer.

The principle of selective nucleotides is disclosed inter alia in EP534858 and in Vos *et al.,* Nucleic Acid Research, 1995, vol. 23, 4407-44014 albeit in a different context, i.e. DNA fingerprinting. The selective nucleotides are complementary to the nucleotides in the (ligated) probes that are located adjacent to the primer binding sequence. The selective nucleotides generally do not form part of the region in the (ligated) probes that is depicted as the primer binding sequence. Primers containing selective nucleotide are denoted as +N primers, in which N stands for the number of selective nucleotides present at the 3'-end of the primer. N is preferably selected from amongst A, C, T or G.

N may also be selected from amongst various nucleotide alternatives, i.e. compounds that are capable of mimicking the behaviour of ACTG-nucleotides but in addition thereto have other characteristics such as the capability of improved hybridisation compared to the ACTG-nucleotides or the capability to modify the stability of the duplex resulting from the hybridisation. Examples thereof are PNA's, LNA's, inosine etc. When the amplification is performed with more than one primer, such as with PCR using two primers, one or both primers can be equipped with selective nucleotides. The number of selective nucleotides may vary, depending on the species or on other particulars determinable by the skilled man. In general the number of selective nucleotides is not more than 10, but at least 5, preferably 4, more preferably 3, most preferred 2 and especially preferred is 1 selective nucleotide.

A +1 primer thus contains one selective nucleotide; a +2 primer contains 2 selective nucleotides etc. A primer with no selective nucleotides (i.e. a conventional primer) can be depicted as a +0 primer (no selective nucleotides added). When a specific selective nucleotide is added, this is depicted by the notion +A or +C etc.

By amplifying a set of connected probes with a selective primer, a subset of connected probes is obtained, provided that the complementary base is incorporated at the appropriate position in the design of the probes that are supposed to be selectively amplified using the selective primer. Using a +1 primer, for example, the multiplex factor of the amplified mixture is reduced by a factor 4 compared to the mixture of connected probes prior to amplification. Higher reductions can be achieved by using primers with multiple selective nucleotides, i.e. 16 fold reduction of the original multiplex ratio is obtained with 2 selective nucleotides etc.

When an assay is developed which, after ligation, is to be selectively amplified, it is preferred that the probe contains the complementary nucleotide adjacent to the primer binding sequence. This allows for pre-selection of the ligated probe to be selectively amplified.

The use of selective primers in the present invention has proven to be advantageously when developing ligation based assays with high multiplex ratios of which subsequently only a specific part needs to be analyzed, resulting in further cost reduction of the ligation reaction per datapoint. By designing primers together with adjacent selective nucleotides, the specific parts of the sample that are to be amplified separately can be selected beforehand.

One of the examples in which this is useful and advantageous is in case of analysis of samples that contain only minute amounts of DNA and/or for the identification of different (strains of) pathogens. For example, in an assay directed to the detection of various strains of anthrax (*Bacillus anthracis*)*,* for each of the strains a pair of representative probes is designed. The detection of the presence, absence or amount of this pair (or a characterizing portion thereof) after the hybridisation and ligation steps of the method of the invention may serve as an identification of the strain concerned. The selective amplification with specifically designed primers (each selective primer is linked to a specific strain) can selectively amplify the various strains, allowing their identification. For instance, amplification with an +A primer selectively amplifies the ligated probes directed to imaginary strain X where a +G primer selectively amplifies the ligated probes directed to imaginary strain Y. If desired, for instance in the case of small amounts of sample DNA, an optional first amplification with a +0 primer will increase the amount of ligated probes, thereby facilitating the selective amplification.

For example, a universal primer of 20 nucleotides becomes a selective primer by the addition of one selective nucleotide at its 3' end, the total length of the primer now is 21 nucleotides. Alternatively, the universal primer can be shortened at its 5' end by the number of selective nucleotides added. For instance, adding two selective nucleotides at the 3'end of the primer sequence can be combined with the absence (or removal) of two nucleotides from the 5'end of the universal primer, compared to the original universal primer. Thus a universal primer of 20 nucleotides is replaced by a selective primer of 20 nucleotides. These primers are depicted as 'nested primers' throughout this application. The use of selective primers based on universal primers has the advantage that amplification parameters such as stringency and temperatures may remain essentially the same for amplification with different selective primers or vary only to a minor extent. Preferably, selective amplification is carried out under conditions of increased stringency compared to non-selective amplification. With increased stringency is meant that the conditions for annealing the primer to the ligated probe are such that only perfectly matching selective primers will be extended by the polymerase used in the amplification step. The specific amplification of only perfectly matching primers can be achieved in practice by the use of a so-called touchdown PCR profile wherein the temperature during the primer annealing step is stepwise lowered by for instance 0.5 °C to allow for perfectly annealed primers. Suitable stringency conditions are for instance as described for AFLP amplification in EP 534858 and in Vos et al., Nucleic Acid Research, 1995, vol. 23, 4407-44014. The skilled man will, based on the guidance find ways to adapt the stringency conditions to suit his specific need without departing from the gist of the invention.

One of the further advantages of the selective amplification of ligated probes is that an assay with a high multiplex ratio can be adapted easily for detection with methods or on platforms that prefer a lower multiplex ratio.

One of many examples thereof is the detection based on length differences such as electrophoresis and preferably capillary electrophoresis such as is performed on a MegaBACE or using nano-technology such as Lab-on-a-Chip.

### Amplification

In step (i) of the method of the invention, the connected probes are amplified to produce an amplified sample comprising amplified (detectable) connected probes (amplicons) that are representations of the target nucleotide sequence by any suitable nucleic acid amplification method known in the art. Nucleic acid amplification methods usually employ one or two primers, dNTP's, and a (DNA) polymerase. A preferred method for amplification is PCR. "PCR" or "Polymerase Chain Reaction" is a rapid procedure for in vitro enzymatic amplification of a specific DNA segment. The DNA to be amplified is denatured by heating the sample. In the presence of DNA polymerase and excess deoxynucleotide triphosphates, oligonucleotides that hybridise specifically to the target sequence prime new DNA synthesis. It is preferred that the polymerase is a DNA polymerase that does not express strand displacement activity or at least not significantly. Examples thereof are Amplitaq and Amplitaq Gold (supplier Perkin Elmer) and Accuprime (Invitrogen). One round of synthesis results in new strands of determinate length, which, like the parental strands, can hybridise to the primers upon denaturation and annealing. The second cycle of denaturation, annealing and synthesis produces two single-stranded products that together compose a discrete double-stranded product, exactly the length between the primer ends. This discrete product accumulates exponentially with each successive round of amplification. Over the course of about 20 to 30 cycles, many million-fold amplification of the discrete fragment can be achieved. PCR protocols are well known in the art, and are described in standard laboratory textbooks, e.g. Ausubel *et al.,* Current Protocols in Molecular Biology, John Wiley & Sons, Inc. (1995). Suitable conditions for the application of PCR in the method of the invention are described in EP-A 0 534 858 and Vos *et al.* (1995; Nucleic Acids Res.23: 4407-4414), where multiple DNA fragments between 70 and 700 nucleotides and containing identical primer-binding sequences are amplified with near equal efficiency using one primer pair. In certain embodiments, the polymerase is inactivated after amplification.

Other multiplex and/or isothermal amplification methods that may be applied include e.g. Rolling circle amplification, LCR, self-sustained sequence replication (3SR), Q-β-replicase mediated RNA amplification, or strand displacement amplification (SDA). In some instances, this may require a different design of the probes and primers without departing from the gist of the invention.

### Amplicons

The term 'amplicon' as used herein refers to the product of the amplification step of the connected probes. The term 'amplicon' as used herein thus refers to an amplified connected probe. After the ligation step wherein the two target specific sections are connected by mean of a ligase, the connected or ligated probe is combined with one or more primers and a polymerase and amplified to produce amplicons. The ligated probe, the primers, the polymerase and/or other parameters and variables are such that the amplification results in amplified linear representations of the connected probe.

Preferably an amplicon is a monomeric representation of the amplified connected probe. In certain embodiments, the amplicon comprises and preferably consists of the nucleotides of the first and optional second primer and the identifier that is located in-between. The various embodiments of the present invention will provide further detail in this respect.

### Detection

The amplicons of the present invention can be detected on a suitable detection platform. The discrimination between amplicons derived from different target sequences can be based on length, sequence or mass as the primary parameter. Detection of the (labelled) samples is performed by a detector to result in detection data. The detector is of course dependent on the general system on which the separation is carried out (length, mass or sequence or a combination thereof) but is, if applicable, also depending on the label that is present on the primer, such as a fluorescent or a radioactive label.

Examples of suitable detection platforms are length based detection platforms, sequence based detection platforms and mass based detection platforms.

### Length based detection

One of many examples of length based detection is the detection based on electrophoresis (capillary electrophoresis, slab-gel electrophoresis, fixed detector-continuous gel-electrophoresis) and preferably capillary electrophoresis such as is performed on MegaBACE equipment available from Amersham Biosciences, or using nano-technology such as Lab-on-a-Chip or other micro-eluidic devices. The difference in length of the amplicon being detected can be provided by the use of one or more identifiers.

The amplicons in a sample are preferably analysed on an electrophoretic device. The electrophoretic device preferably separates the different amplicons in an amplified sample on the basis of length (mobility), after which the separated amplicons may be detected as described herein. The electrophoretic device preferably is a multichannel device in which multiple samples are electrophoresed in multiple channels, preferably in parallel. The electrophoretic device has an application location (per channel) for application (loading) of the amplified sample to be electrophoresed, a separation area over which the fragments in the sample migrate by electrophoresis, and preferably also a detection device located at a detection location distal from the application location. The detection device will usually comprise a photomultiplier for the detection of fluorescence, phosphorescence or chemiluminescence. Alternatively, in the case of gel-electrophoresis, the separated fragments may be detected in the gel e.g. by autoradiography or fluorography.

### Length discrimination

To discriminate between different target sequences in the sample preferably a difference in length of the respective corresponding amplicons is used. By separating the amplicons based on length, the presence of the corresponding target sequences in the sample can be determined. Accordingly, in a preferred embodiment of the present invention, the discrimination between amplicons derived from different target sequences in a sample is based on a length difference between the respective amplicons corresponding to different target sequences in a sample or amplified sample.

Preferably, the length difference is provided by the length of the identifier sequence(s) in the oligonucleotide second probes of the invention. By including in the second oligonucleotide probe of the pair of probes of the invention an identifier of a pre-determined length, the length of each connected probe in an amplified sample can be controlled such that an adequate discrimination based on length differences of the obtained amplicons is enabled. The length differentiation between amplicons obtained from target sequences in the sample is preferably chosen such that the amplicons can be distinguished based on their length. This is accomplished by using identifier sequences that result in length differences of the amplicons that may be distinguished on electrophoretic devices. Thus, from the perspective of resolving power, the length differences between the different amplified connected probes as may be caused by their identifiers, are as large as possible. However, for several other important considerations, as noted hereinbefore, the length differences between the different amplicons is preferably as small as possible: (1) the upper limit that exists in practice with respect to the length of chemically synthesised probes of about 100-150 bases at most; (2) the less efficient amplification of larger fragments; and (3) the increased chances for differential amplification efficiencies of fragments with a large length variation. Preferably the length differences between the sequences to be determined and provided by the identifiers are at least sufficient to allow discrimination between essentially all amplicons derived from one sample. By definition, based on chemical, enzymatic and biological nucleic acid synthesis procedures, the minimal useable size difference between different amplicons in an amplified sample is one base, and this size difference fits within the resolving power of most electrophoresis devices, especially in the lower size ranges. Thus based on the above it is preferred to use multiplex assays with amplification products with differ in length by a single base(pair). In a preferred embodiment, the length difference between different amplicons in an amplified sample is at least two nucleotides. In a particularly preferred embodiment of the invention the amplicons corresponding to different target sequences in a sample have a length difference of two nucleotides.

It should be noted that length based discrimination is, at a molecular level, mostly based on differences in mobility. Hence a length differences can be seen as a mobility difference. The identifier then serves to impart a distinguishable mobility difference, in combination with the other elements of the amplicon. This means that difference in length can be replaced by differences in mobility such are known as mobility modifiers, polymers (such as ethylene glycol oligomers) or other compounds (modified nucleotides etc.) that influence the mobility of a target sequences. Another example is a biotin label that does not add to the length in nucleotides or base pairs of the amplicon or the (ligated) probe, but nevertheless functions as a mobility modifier. This variation is comprised in the present invention. It should be noted that throughout this application, whenever length differences are discussed, mobility differences are explicitly included. Thus, when a length difference of two nucleotides is discussed, a mobility difference corresponding to two nucleotides is included. The use of mobility modifiers may even lower the detection limit of one basepair difference as the currently available technology distinguishes already below the one-basepair level (i.e. 0.1 basepair).

### Length and label

Throughput can be increased by the use of multiple labelled primers. One of the problems associated with the use of different labels in one sample is cross talk or residual cross talk. Cross talk or residual cross talk, as used herein, refers to the overlap between the emission spectra of different (fluorescent) labels. For instance when fluorescent dyes are used, each dye has a different emission (and absorption) spectrum. In case of two dyes in one sample, these spectra can overlap and may cause a disturbance of the signal, which contravenes the quality of the data obtained. Particularly when two nucleotide fragments to be detected in a sample are labelled with a different label and one of the fragments is present in an abundant amount whereas the other is present only in minute amounts, residual cross talk can cause that the measured signal of the fragment that is present in only minute amounts is mostly derived from the emission of another label with an overlapping emission spectrum that is abundantly contained in a fragment with identical size of another sample. The reciprocal effect of the other dye may also occur but in this example its effect is probably less because of the abundance differences between the amplicons labelled with the respective dyes.

Chehab *et al.* (Proc. Natl. Acad. Sci. USA, 86:9178-9182 (1989) have attempted to discriminate between alleles by attaching different fluorescent dyes to competing alleles in a single reaction tube by selecting combinations of labels such that the emission maximum of one dye essentially coincides with the emission minimum of the other dye. However, at a certain wavelength at which one dye expresses an absorption maximum, there is always also some remaining absorption from another dye present in the sample, especially when the sample contains multiple dyes.

This route to multiplex analysis was found to be limited in scale by the relatively few dyes that can be spectrally resolved. One of the major problems with the use of multiple dyes is that the emission spectra of different fluorescent labels often overlap. The resulting raw data signals have to be corrected for the contribution of similar size fragments that are detected simultaneously and are labelled with another fluorescent dye by a process called cross-talk correction. Cross-talk correction is commonly carried out by mathematical means, based on the known theoretical absorption spectra for both dyes, after "raw" data collection from the detection device. Mathematical correction is based on theoretical spectra and ignores that emission spectra of labels are sensitive and often affected by the composition of the detection sample. These sensitivities can affect the brightness and/or the wavelength of the emission. This means that parameters such as pH, temperature, excitation light intensity, non-covalent interactions, salt concentration and ionic strength strongly influence the resulting emission spectrum. In particular, it is known that the presence of residual salts in a sample affects the fluorescence signal emitted by the dye and is a critical factor in case of detection by capillary electrophoresis using electrokinetic injection because it then also affects the injection efficiency. Thus, spectral overlap is a potential source of error that negatively impacts on data quality in case of multiplex detection using different fluorescent dyes.

The present invention provides for a solution to this problem such that two (or more) labels with overlapping spectra can be used in the same sample without significantly affecting data quality. By a predetermined combination of length differences and labels, an increase in the number of target nucleotide sequences that can be detected in sample is obtained while the quality of the data remains at least constant. In a preferred embodiment of the invention, spectral overlap between two differently labelled sequences is reduced by the introduction of a length difference between the two sequences. This label-related length difference can be provided for by the length of the identifier sequence as described herein. The number of different labels that can be used in the same sample in the present method is at least two, preferably at least three, more preferably at least four. The maximum number of labels is functionally limited by the minimum of spectral overlap that remains acceptable, which for most applications typically amounts to less than 15 percent of the true signal, preferably less than 10 percent, more preferably lees than 5 percent and most preferably less than 1 percent of the true signal.

In order to avoid the potential influence of residual cross-talk on the data quality in case different samples are labelled with multiple fluorescent dyes with overlapping emission spectra and fragments with identical length are detected simultaneously in the same run, in a particular preferred embodiment it is preferred to choose the identifier sequences such that amplicons differ by at least two base pairs (nucleotides) within a multiplex set and differ by a single base pair between multiplex sets labelled with the different dyes that have overlapping spectra. By doing so, the length of the fragments labelled with the respective dyes can be chosen such that the potential influence of residual cross-talk on the quality of the data is circumvented because unique combinations of fragments size and labelling dye are defined.

A particular preferred embodiment of the invention is directed to a method in which a sample comprising amplicons is derived from a multiplicity of target sequences. These amplicons are differently labelled, thereby defining groups of amplicons carrying the same label. Within each group, the identifier provided for a length difference of at least two, preferably two nucleotides. Between two groups with labels having spectral overlap, the identifier provides a length difference of one nucleotide, effectively resulting in one group having an even number of nucleotides and one group having an odd number of nucleotides as described above.

In one aspect the present invention pertains to a method for the improved discrimination and detection of target sequences in a sample, comprising providing at least a two or more groups of oligonucleotide probes, wherein the amplicons obtained with different groups of oligonucleotide probes have different labels, wherein substantially each amplicon within a group has the same label, wherein within a group of identically labelled amplicons a length difference is provided between each identically labelled probe within that group, wherein between the first and second group an additional length difference is provided such that each amplicon in the amplified sample is characterised by a combination of length of the sequence and the label.

In a preferred embodiment of the method of the invention, at least two groups of pairs of first and second probes are provided to a sample, whereby each group of second oligonucleotide probes has tag sequences with at least one group specific primer-binding site. The connected probes of each group are amplified from a primer pair wherein at least one of the first and (optional) second primer is complementary to the group specific primer-binding site, and whereby at least one of the first and second primers of a group comprises a group specific label. In each group, an amplicon corresponding to a target sequence in the sample differs in length from an amplicon corresponding to a different target sequence in the sample. The group specific labels are preferably such that the detection device can distinguish between the different group specific labels. The length difference is preferably provided by the length of the identifier sequence. Preferably in this embodiment of the method of the invention, a first part of the group has amplicons having an even number of nucleotides and a second part of the group has amplicons having an odd number of nucleotides. Preferably, the groups of amplicons having an even number of nucleotides and the groups of amplicons having an odd number of nucleotides are labelled with (fluorescent) labels, which have the least overlap in their emission spectra. Thus, two groups of amplicons, each group having an odd number of nucleotides are labelled with labels which have the least overlap in their emission spectra. The same holds for two groups of amplicons, each group having an even number of nucleotides. Two groups of amplicons, one group having an odd number of nucleotides and the other group having an even number of nucleotides are labelled with labels that have a larger overlap in their emission spectra. The relative notions as used herein of 'the least overlap in their emission spectra' and 'have a larger overlap in their emission spectra' refer to a group of labels from which a selection of the labels can be made for use in the present invention. This group of labels may depend on the detection platform used to other factors such as those disclosed herein before. In a particularly preferred embodiment of this method, a first and second groups of amplicons having an even number of nucleotides are produced and a third and fourth group of amplicons having an odd number of nucleotides are produced and whereby the first and second group are labelled with FAM and NED, respectively, and the third and fourth group are labelled with (ET-)ROX and either JOE or HEX, respectively; or *vice versa,* whereby the first and second group are labelled with (ET-)ROX and either JOE or HEX, respectively, and the third and fourth group are labelled with FAM and NED, respectively. Thus, in these embodiments, the fluorescent labels are chosen such that the groups of amplicons that co-migrate, because they both contain fragments with either even or odd numbers of nucleotides, have labels which have the least overlap in their emission spectra, thereby avoiding as much as possible cross-talk in the detection of amplicons in different groups (see also below).

In a preferred embodiment to avoid cross-talk it is therefore desirable to combine a difference in length with a different label when analysing a set of amplicons in such a way that the influence of spectral overlap on the data quality is avoided by length differences between the amplicons labelled with the dyes that have overlapping emission spectra.

It is preferred that in each sample amplicons derived from each target sequence differ from any other amplicons in the sample in length, and/or in the label or, preferably in the combination of the length and the label. To provide for an adequate separation of the amplicons of different length it is preferred that the length difference between two different amplicons is at least two nucleotides, preferably two. When detecting polymorphisms it is preferred that the difference in length between two or more (SNP) alleles of the polymorphism is not more than two, thereby ensuring that the efficiency of the amplification is similar between different alleles or forms of the same polymorphism. This implies that preferably both alleles are amplified with the same pair of primers and hence will be labelled with the same dye.

Certain embodiments are directed to the detection of different alleles of one locus. Certain embodiments are directed to the detection of different alleles of a multiplicity of loci. In certain embodiments, the distribution between odd/even lengths within a group can be designed in the following way. Two loci L1, L2 are each represented by two alleles A11, A12 for L1 and A21, A22 for L2. The lengths of the various alleles (or amplicons representing those alleles) is such that A11>A12>A21>A22; A12-A11=2; A22-A21=2; A12-A21=3. Between groups G1 and G2 carrying labels that may have an overlap in their spectra there can be a length difference of 1 nucleotide. Thus G1(A11)-G2(A11)=1, hence the group starts with either an even or an uneven length.

This distribution has some significant advantages compared to the more densely packed distribution disclosed herein. It is known that due to conformational differences different sequences of identical length generally differ in their electrophoretic mobility. When there is only a difference in length of one nucleotide, this may cause overlap between the peaks if the sequences are of a very different mobility. For instance the difference in mobility between two alleles of one locus (A11, A12), will be less than the difference in mobility between two alleles from different loci (A12, A21). When there is a significant difference in mobility between A12 and A21, this may lead to unreliable detection. By creating length distributions as herein disclosed this can be avoided. The lower throughput is then weighed against the reliability of the detection.

The problem of the overlap between the spectra of the different labels is then adequately avoided. This is schematically depicted in Table A.

**Table A:**

| Alternative distribution scheme of labels and lengths of probes. | | | | |
|---|---|---|---|---|
| Length | Group 1-Label 1 | Group 2-Label 2 | Group 3-Label 3 | Group 4-Label 4 |
| N | G1A11 | | G3A11 | |
| N+1 | | G2A11 | | G4A11 |
| N+2 | G1A12 | | G3A12 | |
| N+3 | | G2A12 | | G4A12 |
| N+4 | | | | |
| N+5 | G1A21 | | G3A21 | |
| N+6 | | G2A21 | | G4A21 |
| N+7 | G1A22 | | G3A22 | |
| N+8 | | G2A22 | | G4A22 |
| N+9 | | | | |
| N+10 | G1A31 | | G3A31 | |
| N+11 | | G2A31 | | G4A31 |
| N+12 | G1A32 | | G3A32 | |
| N+13 | | G2A32 | | G4A32 |
| N+14 | | | | |
| N+15 | G1A41 | | G3A41 | |
| N+16 | | G2A41 | | G4A41 |
| N+17 | G1A42 | | G3A42 | |
| N+18 | | G2A42 | | G4A42 |

In an embodiment of the present invention there is provided between the amplicons within one group, a length difference of alternating two and three nucleotides, i.e. 0, 2, 5, 7, 10, 12 etc. The other group then has a length difference of 1, 3, 6, 8, 11, 13 etc. Based on the information disclosed herein, the skilled man may determine other ways of varying length differences within a range.

### Size ladder

The sample can be supplied with a nucleotide fragment size standard comprising one or more nucleotide fragments of known length. Methods of preparing and using nucleotide size standards are well known in the art (see e.g. Sambrook and Russel, 2001, *supra*)*.* Such a size standard forms the basis for appropriate sizing of the amplicons in the sample, and hence, for the proper identification of the detected fragment. The size standard is preferably supplied with every sample and/or with every injection. A size standard preferably contains a variety of lengths that preferably spans the entire region of lengths to be analysed. In a particular embodiment of the invention, it is considered advantageously to add flanking size standards from which the sizes of the amplicons can be derived by interpolation. A flanking size standard is a size standard that comprises at least two labelled oligonucleotide sequences of which preferably one has a length that is at least one base shorter than the shortest amplicon and preferably one that is a least one base longer than the longest amplicon to allow interpolation and minimise the introduction of further length variation in the sample. A preferred flanking size standard contains one nucleotide that is one nucleotide shorter the shortest amplicon and one that is a least one base longer than the longest amplicon and is labelled with at least one dye that is identical to the label used for labelling the amplicons contained in the sample.

A convenient way to assemble a suitable size standard is by (custom) chemical synthesis of oligonucleotides of the appropriate lengths, which are end-labelled with a suitable label. The size standard is applied with every consecutively applied sample to serve as local size references to size the loaded sample fragments. The size standard may be applied in the same channel or lane of the electrophoretic device as the sample to be analysed, i.e. together with the sample, or may be applied in a parallel channel or lane of a multichannel/lane device. The flanking size standard can be labelled with any of the labels used in the method. If the size standard is applied in the same channel of the device, the fragments of the standard are preferably labelled with a label that can be distinguished from the labels used for the detection of the amplicons in a sample.

### Sequence based detection

Examples of sequence based detection platforms are solid phase and fluid phase microarrays. Preferably, uniquely addressable arrays are used wherein the probe contains a unique sequence (such as a ZIP sequence) as (part of) the identifier, thereby providing that the amplicon will hybridise to a predetermined spot on the array wherein the complementary ZIP sequence is located (cZIP). Array-based detection methods are commonplace nowadays and the technology is widely spread, allowing the skilled man to create a suitable array for the detection of the amplicons of the present invention.

One embodiment of the invention using sequence based detection relates to a method for determining the presence, absence or amount of a target sequence in a nucleic acid sample, wherein the presence, absence or amount of the target sequence is determined by an oligonucleotide ligation assay in combination with a detection method based upon sequence and wherein each target sequence in the sample is represented by an identifier and detection of the target sequences is based on the detection of the presence or the absence of a fragment comprising said identifier.

Examples of suitable array based detection methods are for instance WO 97/27317, WO 97/22720, WO 97/43450, EP 0 799 897, EP 0 785 280, WO 97/31256, WO 97/27317, WO 98/08083, and the Genechips array, the Affymetrix DNA chip and the VLSIPSTM array. Especially suitable and preferred detection platforms for the assay of the present invention are arrays described in inter alia WO9902266, EP1050588, WO0119517, WO02072263, WO02072268, WO02072266, the so-called Pam arrays.

### Mass based detection

An example of mass based platforms is MALDI-TOF. The analytes to be detected each have a different mass. This can be achieved for instance by the incorporation of a identifier sequence comprising a restriction site in the second probe. When the amplicons are restricted prior to detection, a set of fragments/oligonucleotides are obtained, each having a different mass that is associated with the presence, absence or amount of a target sequence in the sample.

One embodiment of the invention using mass based detection relates to a method for determining the presence, absence or amount of a target sequence in a nucleic acid sample, wherein the presence, absence or amount of the target sequence is determined by an oligonucleotide ligation assay in combination with a detection method based upon molecular mass and wherein each target sequence in the sample is represented by an identifier and detection of the target sequences is based on the detection of the presence or the absence of a fragment comprising said identifier. This method has also been disclosed in WO03/030163 by applicant.

It is clear from the description of the present invention in all its embodiments that a versatile and flexible method is provided for the generation of fragments of precisely controlled length, mass or nucleotide sequence with a predictable behavior on a variety of detection platforms that are uniquely linked to the occurrence of a ligation event that is coupled to the presence, absence or amount of a target sequence in a sample. The present invention provides for a flexible and scaleable method for the multiplexed detection of target sequences varying between large scale (hundreds of thousands, medium scale (hundreds) and small scale (up to fifty - hundred).

The method according to the invention allows for the analysis of a multiplicity of target sequences thereby significantly increasing the throughput of the number of samples that can be analysed. "Throughput" as used herein, defines a relative parameter indicating the number of samples and target sequences that can be analysed per unit of time.

### Pooling

In a variant of the technology, the starting (DNA) material of multiple individuals are pooled such that less detection samples containing this material are loaded on the detection device, This can be advantageous in the case of Linkage Disequilibrium (LD mapping) when the objective is to identify amplicons (such as those representing SNP alleles) that are specific for a particular pool of starting samples, for example pools of starting material derived from individuals which have different phenotypes for a particular trait.

### Application

One aspect of the invention pertains to the use of the method in a variety of applications. Application of the method according to the invention is found in, but not limited to, techniques such as genotyping, transcript profiling, genetic mapping, gene discovery, marker assisted selection, seed quality control, hybrid selection, QTL mapping, bulked segregant analysis, DNA fingerprinting and microsatellite analysis. Another aspect pertains to the simultaneous high throughput detection of the quantitative abundance of target nucleic acids sequences. This approach is commonly known as Bulked Segregant Analysis (BSA).

### Detection of single nucleotide polymorphisms

One particular preferred application of the method according to the invention is found in the detection of single nucleotide polymorphisms (SNPs). A first oligonucleotide probe (and preferably the first probe) of the pair according to the invention comprises a part that is complementary to a part of the target sequence that is preferably located adjacent to the polymorphic site, i.e. the single polymorphic nucleotide. A second oligonucleotide probe (and preferably the second probe) of the pair according to the invention is complementary to the part of the target sequence such that its terminal base is located at the polymorphic site, i.e. is complementary to the single polymorphic nucleotide. If the terminal base is complementary to the nucleotide present at the polymorphic site in a target sequence, it will anneal to the target sequence and will result in the ligation of the two probes. When the end-nucleotide, i.e. the allele-specific nucleotide does not match, no ligation or only a low level of ligation will occur and the polymorphism will remain undetected.

When one of the target sequences in a sample is derived from or contains a single nucleotide polymorphism (SNP), in addition to the probes specific for that allele, further probes can be provided that not only allow for the identification of that allele, but also for the identification of each of the possible alleles of the SNP (co-dominant scoring). To this end a combination of types of probes can be provided: one type probe that is the same for all alleles concerned and one or more of the other type of probe which is specific for each of the possible alleles. These one or more other types of probes contain the same complementary sequence but differ in that each contains a nucleotide, preferably at the end, that corresponds to the specific allele. The allele specific probe can be provided in a number corresponding to the number of different alleles expected. The result is that one SNP can be characterised by the combination of one type of probe with four other type (allele-specific) probes, identifying all four theoretically possible alleles (one for A, T, C, and G), by incorporating identifier sequences of different lengths (preferred) or different labels into the allele specific probes.

In a particular embodiment, preferably directed to the identification of single nucleotide polymorphisms, the first oligonucleotide probe of the pair according to the invention is directed to a part of the target sequence that does not contain the polymorphic site and the second oligonucleotide probe of the pair according to the invention contains, preferably at the end distal from the tag section, one or more nucleotide(s) complementary to the polymorphic site of interest. After ligation of the adjacent probes, the connected probe is specific for one of the alleles of a single nucleotide polymorphism. To identify the allele of the polymorphic site in the target sequence, a pair of oligonucleotide probes can be provided wherein one first probe is provided and one or more second probes (in this case the pair of probes may contain more than two probes). Each second probe then contains a specific nucleotide at the end of the complementary sequence, preferably the 3'-end, in combination with a known length of the identifier. For instance, in case of an A/C polymorphism, the second probe can contain a specific nucleotide T in combination with a identifier length of 2 nucleotides and another second probe for this polymorphism combines a specific nucleotide G with a identifier length of 0. This creates a length difference of the resulting amplicons of 2 nucleotides. In case the detection of the presence and/or the absence of all four theoretically possible nucleotides of the polymorphic site is desired, the identifier-specific nucleotide combination can be adapted accordingly. In a sample containing multiple target sequences, amplified with the same pair of amplification-primers (and hence label) or with multiple pairs of amplification primers with labels that have overlapping emission spectra, the identifier lengths are chosen such that all connecter probes and the resulting amplicons are of a unique length. In a preferred embodiment this principle can be extended to at least ten loci with at least two alleles per locus.

### Detection of specific target sequence

The target sequence contains a known nucleotide sequence derived from a genome. Such a sequence does not necessarily contain a polymorphism, but is for instance specific for a gene, a promoter, an introgression segment or a transgene or contains information regarding a production trait, disease resistance, yield, hybrid vigour, is indicative of tumours or other diseases and/or gene function in humans, animals and plants. To this end, the complementary parts of the first probe and the second probe are designed to correspond to a, preferably unique, target sequence in the genome or the RNA, associated with the desired information. The complementary parts in the target sequence are located adjacent to each other. In case the desired target sequence is present in the sample, the two probes will anneal adjacently and after the subsequent steps comprising ligation and amplification, can be detected.

### Detection of AFLP markers

AFLP, its application and technology is described in Vos *et al.,* Nucleic Acids Research, vol. 23, (1995), 4407-4414 as well as in EP-A 0 534 858 and US 6045994, all incorporated herein by reference. For a further description of AFLP, its advantages, its embodiments, its techniques, enzymes, adapters, primers and further compounds, tools and definitions used, explicit reference is made to the relevant passages of the publications mentioned hereinbefore relating to AFLP. AFLP and its related technology is a powerful DNA fingerprinting technique for the identification of for instance specific genetic markers (so-called AFLP-markers), which can be indicative of the presence of certain genes or genetic traits or can in general be used for comparing DNA, cDNA or RNA samples of known origin or restriction pattern. AFLP-markers are in general associated with the presence of polymorphic sites in a nucleotide sequence to be analysed. Such a polymorphism can be present in the restriction site, in the selective nucleotides, for instance in the form of indels or substitutions or in the rest of the restriction fragment, for instance in the form of indels or substitutions. Once an AFLP marker is identified as such, the polymorphism associated with the AFLP-marker can be identified and probes can be developed for use in the ligation assay of the present invention.

In another aspect, the present invention pertains to a first nucleic acid probe as described herein comprising and preferably consisting of a part that is capable of hybridising to a first part of a target sequence. The invention also pertains to a second nucleic acid probe as described herein comprising a part that is capable of hybridising to a second part of the target sequence, and preferably comprising a primer-binding sequence and/or an identifier. The invention also pertains to a pair of probes, preferably comprising a first and second probe. The invention also pertains to a pair of probes, preferably comprising a first and second probe.

The invention in a further aspect pertains to the use of a pair of probes in the analysis of at least one nucleotide sequence and preferably in the detection of a single nucleotide polymorphism, wherein the pair further comprises at least one additional probe that contains a nucleotide that is complementary to the known SNP allele. Preferably the pair comprises a probe for each allele of a specific single nucleotide polymorphism. The use of a pair of probes is further preferred in a method for the high throughput detection of single nucleotide polymorphisms wherein the length or the presence of the identifier in the probe is specific for a locus-allele combination of a single nucleotide polymorphism in a target sequence.

Another aspect of the invention relates to the primers and more in particular to the pair of primers comprising a first primer and one or more second primers, wherein each second primer contains a detectable label and which second primer comprises a nucleotide sequence that is specific for said label.

The present invention also finds embodiments in the form of kits. Kits according to the invention are for instance kits comprising (pairs of) probes suitable for use in the method as well as a kit comprising primers or selective primers, further a combination kit, comprising primers and probes, preferably all suitably equipped with enzymes buffers etcetera, is provided by the present invention.

The invention also relates to the use of a pair of probes or two or more pairs of probes according to the invention in the detection or determination of the presence, absence or amount of a target sequence in at least one sample.

### Description of the Figures

**Figure 1:** Schematic representation of an embodiment of the method of the invention in which exonuclease is used in combination with exonuclease resistant probes. A target sequence (1) is brought into contact with a first probe (2) that contains an exonuclease resistant section (3) and a first target specific section (4). A second probe (5) is provided, comprising a second target specific section (6), a first primer binding site (7), an identifier (8) and a second primer binding site (9). The probes are allowed to anneal to the target sequence. The probes that are annealed adjacent on the target sequence are ligated (match) to form a connected probe (10). If the probes are not annealed adjacent, no ligation occurs (mismatch). In this Figure the orientation of the target sequence is given as 5'-3' (left-right), which determines the orientation of probes, primers and amplicons as well as the orientation of the exonuclease (in this Figure as 3'-5') . As outlined elsewhere, the orientation can be reversed. Exonuclease (11) is provided and the non ligated second probes and/or the target sequence are digested through the action of the exonuclease. The probes that are exonuclease resistant (connected probes and the exonuclease resistant sections of the unligated first and second probes) cannot be digested by the exonuclease (12). The connected probes that are not digested can be amplified using primers (13) to provide amplicons (14). The amplicons can be detected on a suitable platform.

**Figure 2:** Schematic representation of the embodiment wherein the first probe and second probe are directed in a 3' to 5' orientation (left side) and the alternative wherein the probes are directed in a 5' to 3' orientation (right side). The selection of the orientation of the exonuclease (3-5' exonuclease activity or 5'-3' exonuclease activity) may depend on the orientation of the probes, i.e. 3'-5' exonuclease in case of 3'-5' orientation and vice versa. This variation in orientation of the probe versus the target sequence is applicable to all types of probes, whether non-circularizable, circularizable or semi-circularizable.

**Figure 3A:** Schematic representation of an embodiment directed to allele specific detection. The ligation of the allele specific probe leads to the amplicon that is representative (containing the identifier ••) for the presence of the A-allele in the sample. The absence of the amplicon representative (containing the identifier •••) for the C-allele is indicative of the absence of the C-allele in the sample. In Figure 3A, the second probe is the allele specific probe, but in certain embodiments, the first probe may be the allele specific probe.

**Figure 3B:** Schematic representation of the detection of different target sequences in a sample. The presence of the CTA target sequence leads to amplicons that are different (••) from the amplicons obtained from the presence in the sample of the CCG target sequence **(•••••).**

**Figure 4:** schematic representation of the Keylock probe embodiment. The target sequence is brought into contact with a pair of two probes that, in addition to the previously described components of the probes, each comprise a clamp section (15). The clamp sections in the two probes are complementary to each other. These probes exhibit the advantageous hybridisation and ligation characteristics of circularizable or padlock probes, but can be synthesised with higher accuracy due to the relatively shorter length compared to padlock probes.

**Figure 5A**: Schematic representation of a generic oligonucleotide ligation assay (i.e. based on symmetrical probes) wherein, when a first probe and a second probe are annealed to the target sequence, one of the probes contains an overhang and/or overlap (16) at the foreseen point of ligation. The overhang can be removed using an enzyme that cleaves these cleavage structures in highly specific manner after which ligation, amplification and detection can proceed in the conventional manner.

**Figure 5B:** Schematic representation of the embodiment of the present invention (i.e. asymmetric probes) wherein the first or the second probe contains an overhang that can be removed using the enzyme that cleaves these cleavage structures after which ligation, amplification and detection can proceed in the conventional manner.

**Figure 6**: Schematic representation of a embodiment of the present invention based on circularizable probes. Figure 6A (left) represents conventional circularizable probes comprising first (7) and second (9) primer binding sites and an identifier (8). After ligation the circularized probe is amplified resulting in amplicon that contain the ligated target specific sections. In Figure 6B (right) the circularizable probes of the present invention are depicted, comprising the first (7) and second (9) primer binding sites and the identifier (8) in a different orientation compared to the probes of Figure 6A. After ligation, the reaction mixture is treated with an exonuclease to remove any non-ligated (mismatch) probes. Subsequent amplification provides amplicons that do not contain the ligated target specific sections but are correct representations of the target sequence.

### Examples

The invention is now illustrated by means of the following examples. Suitable experimental conditions, in particular relating to ligation, amplification and detection conditions can also be found in WO 03/052140, WO 03/052141, WO 03/052142 and WO 03/30163.

### Example 1. Description of biological materials and DNA isolation

DNA was isolated from leaf material of 4 homozygous tomato lines using methods known *per se,* for instance essentially as described in EP 0 534 858, and stored in 1X TE (10 mM Tris-HCl pH 8.0 containing 1 mM EDTA) solution. Concentrations were determined by UV measurements in a spectrophotometer (MERK) using standard procedures, and adjusted to 100 ng / µl using 1X TE.

### Example 2. Identification of SNPs

The selected SNPs are identified and summarised in Table 1.

### Example 3. Oligonucleotide probe design for oligonucleotide ligation reaction

The oligonucleotide probes (5'-3' orientation) were selected to discriminate the SNP alleles for each of the SNP loci described in Example 2. All the probes are phosphorylated at the 5' end. The sequences are summarised in Tables 2A1 and 2B1 (with clamps) and in Tables 2A2 and 2B2 (without clamps). One group of first probes contains phosphorothioate linkages to render the probes exonuclease resistant (indicated in bold, the three most 3'nucleotides, Table 2A1 and 2A2). Another group of first probes is biotinylated at the 3' end (Table 2B1 and 2B2).

The second probes are provided in both allele specific forms and with an identifier (indicated in bold) generating a length difference of two nucleotides between two alleles for one locus (Table 3).

### Example 5. Design of the PCR amplification primers

Usually the forward primer is labelled. The concentration of the oligonucleotides was adjusted to 50 ng / µl. The sequence of the primers in 5'-3' orientation is depicted in Table 4.

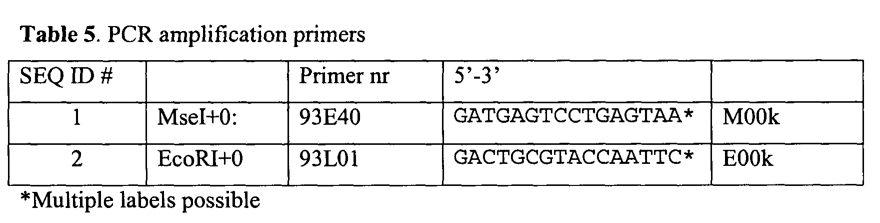

### Example 6. Ligation

4 samples (samples 1-4) of homozygous tomato lines (Example 1) were subjected to a multiplex oligonucleotide ligation reaction using a mixture of 20 probes (2 probes per locus). Conditions used were 1x Taq DNA ligase buffer (NEB), 0.2 U/µl Taq DNA ligase, and 0.05 fmol/µl of each probe in a volume of 10 µl. Ligation was performed in a thermocycler (Perkin Elmer) with the following cycling conditions: 2 minutes at 94 °C + 10*(15 seconds at 94 °C + 60 minutes at 60 °C) + 4 °C continuously. Following ligation, the 10 µl ligation product was diluted with 30 µl 1x Taq DNA ligase buffer.

### Example 7. Removal of unligated probes and target sequences

To 5 µL of the ligation product, 2.5 µL of the exonuclease reaction mix was added (0.033 µL of 10 units/µL Exo I, 0.297 µL SAP dilution buffer, 0.5 µL PCR buffer, 1.92 µL MQ) and 2.5 µL MQ to a total volume of 10 µL. The mixture was incubated by 37 °C for a period varying between 30 minutes and overnight, heated to 80 °C for 10 minutes and kept indefinitely at 4°C or until further use.
Alternatively, 2,5 µL of the ligation product was used, the ligation product was desalted prior to the exonuclease treatment, the PCR and/or the SAP buffer replaced with MQ and/or the amount of EXO1 tenfold increased. Blanks were run for all experiments. In an alternative experiment, 10 µL of exonuclease mix was added to 10 µL of the ligation product for a final concentration of 67 mM Tris-HCL PH 9.0, 50 mM KCL; 6.7 mM MgCl2, 0.05 µg/µLBSA, 035 U/µL Exo I and 035 5 U/µL Exo III and incubated 37 °C for a period varying between 30 minutes and overnight, heated to 80 °C for 10 minutes

For typical biotin purification, the following protocol was used: 2.5 µL streptavidin coated beads (Dynabeads M-280, Dynal) were washed with 100 µL TEX (T10E1+ 0.1% TritinX100). 50 µL beads in TEX were combined with 2.5 µL undiluted ligation product for 30 minutes at RT followed by lye washing (0.1 N NaOH) for denaturation. Washing 1 * with ligation buffer and T10E0.2 and taking up the beads in 10 µL ligation buffer.

### Example 8. Amplification

Ten µl of the reaction product of example 7 was used to perform a PCR using a labelled E00k primer combined with M00k. The E00k primer was labelled with JOE to enable detection on the MegaBACE. Conditions used in the PCR were 30 ng labelled E00k primer and 30 ng M00k primer, 1x Accuprime buffer I, 0.4 ul Accuprime polymerase (Invitrogen) on 10 µl diluted ligation product in a 20 µl PCR reaction. PCR was performed in a thermocycler with the following cycling conditions: 2 minutes at 94 °C + 35 *(15 seconds at 94 °C + 30 seconds at 56 °C + 60 seconds at 68 °C) +4 °C continuously.

PCR product was purified using Sephadex 50 and diluted 80 times with MQ. Diluted PCR product was analysed on the MegaBACE.

### Buffer compositions:

1x Taq DNA ligase buffer
   20 mM Tris-HCl
   25 mM potassium acetate
   10 mM Magnesium acetate
   10 mM DTT
   1 mM NAD
   0.1% Triton X-100
   (pH 7.6@ 25°C)
1xAccuPrime Taq DNApolymerase buffer
   20 mM Tris-HCl (pH8.4)
   50 mM KCl
   1.5 mM MgCl₂
   0.2 mM dGTP, dATP, dTTP and dCTP
   thermostable AccuPrime™ protein
   10% glycerol.

### Example 9. Purification and dilution of amplicons

In case of detection using the MegaBACE 1000 capillary sequencing instrument, desalting and purification of the PCR reactions mixtures was carried in 96-well format, using the following procedure:

### Preparation of the 96-well Sephadex™ purification plates

Dry Sephadex™ G-50 superfine (Amersham Pharmacia Biotech, Uppsala, Sweden) was loaded into the wells of a 96-well plate (MultiScreen®-HV, Millipore Corporation, Bedford, MA, USA), using the 45 microliter column loader (Millipore Corporation) as follows: Sephadex™ G-50 superfine was added to the column loader.

Excess SephadexTM was removed from the top of the column loader with a scraper. The Multiscreen-HV plate was placed upside-down on top of the Column Loader. The Multiscreen-HV plate and the Column Loader were both inverted.

The Sephadex™ G-50 was released by tapping on top or at the side of the Column Loader. Next, the Sephadex™ G-50 was swollen en rinsed as follows: 200 µl Milli-Q water was added per well using a multi-channel pipettor. A centrifuge alignment frame was placed on top of a standard 96-well microplate, the Multiscreen-HV plate was place on top and the minicolumns were packed by centrifugation for 5 min at 900 g.

The 96-well plate was emptied and placed back. Steps 5-7 were repeated once.

200 µl Milli-Q water was added to each well to swell the Sephadex™ G-50 and incubated for 2-3 hours. Occasionally, at this stage the Multiscreen-HV plates with swollen mini-columns of Sephadex™ G-50 superfine were tightly sealed with parafilm and stored a refrigerator at 4 °C until further use. A centrifuge alignment frame was placed on top of a standard 96-well microplate, the Multiscreen-HV plate was placed on top of the assembly and the minicolumns were packed by centrifugation for 5 min at 900 g. The 96-well microplate was removed. The mixtures containing the amplicons were carefully added to the centre of each well. Using the centrifuge alignment frame, the Multiscreen-HV plate was placed on top of a new standard U-bottom microtitre plate and centrifugation was carried out for 5 min at 900 g. The eluate in the standard 96-well plate (approximately 25 µl per well) contains the purified product.

Purified samples were diluted 25-75 fold in Milli-Q water before injection.

### Example 10. Capillary electrophoresis on the MegaBACE

### Preparation of the samples:

A 800-fold dilution of ET-900 Rox size standard (Amersham Biosciences) was made in water. 8 µl diluted ET-900 Rox was added to 2 µl purified sample. Prior to running, the sample containing the sizing standard was heat denatured by incubation for 1 min at 94 °C and subsequently put on ice.

### Detection on the MegaBACE:

MegaBACE capillaries were filled with 1X LPA matrix (Amersham Biosciences, Piscataway, NJ, USA) according to the manufacturer's instructions. Parameters for electrokinetic injection of the samples were as follows: 45 sec at 3 kV. The run parameters were 110 min at 10 kV. Post-running, the cross-talk correction, smoothing of the peaks and cross-talk correction was carried out using Genetic Profiler software, version 1.0 build 20001017 (Molecular Dynamics, Sunnyvale, CA, USA), and electropherograms generated.

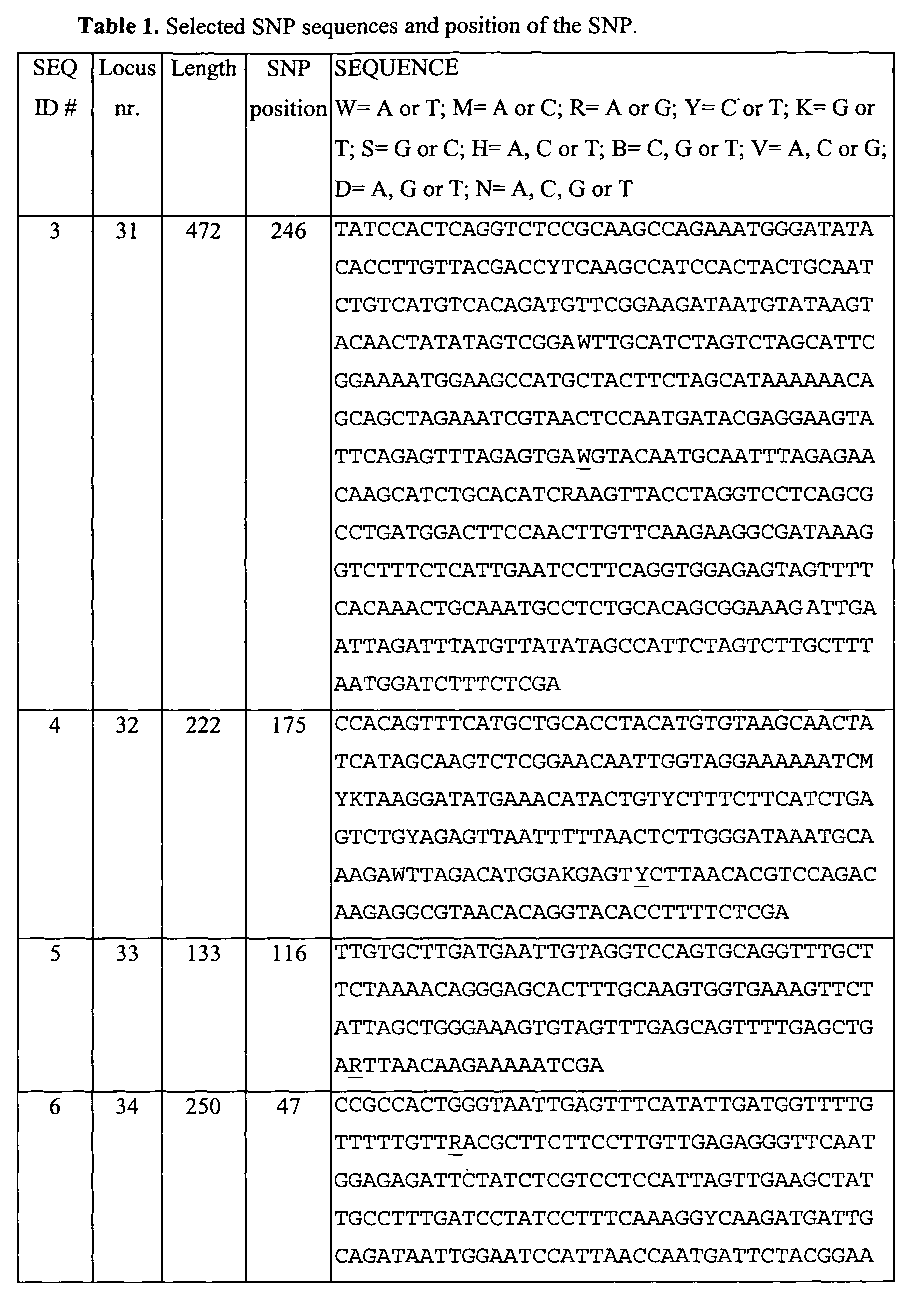

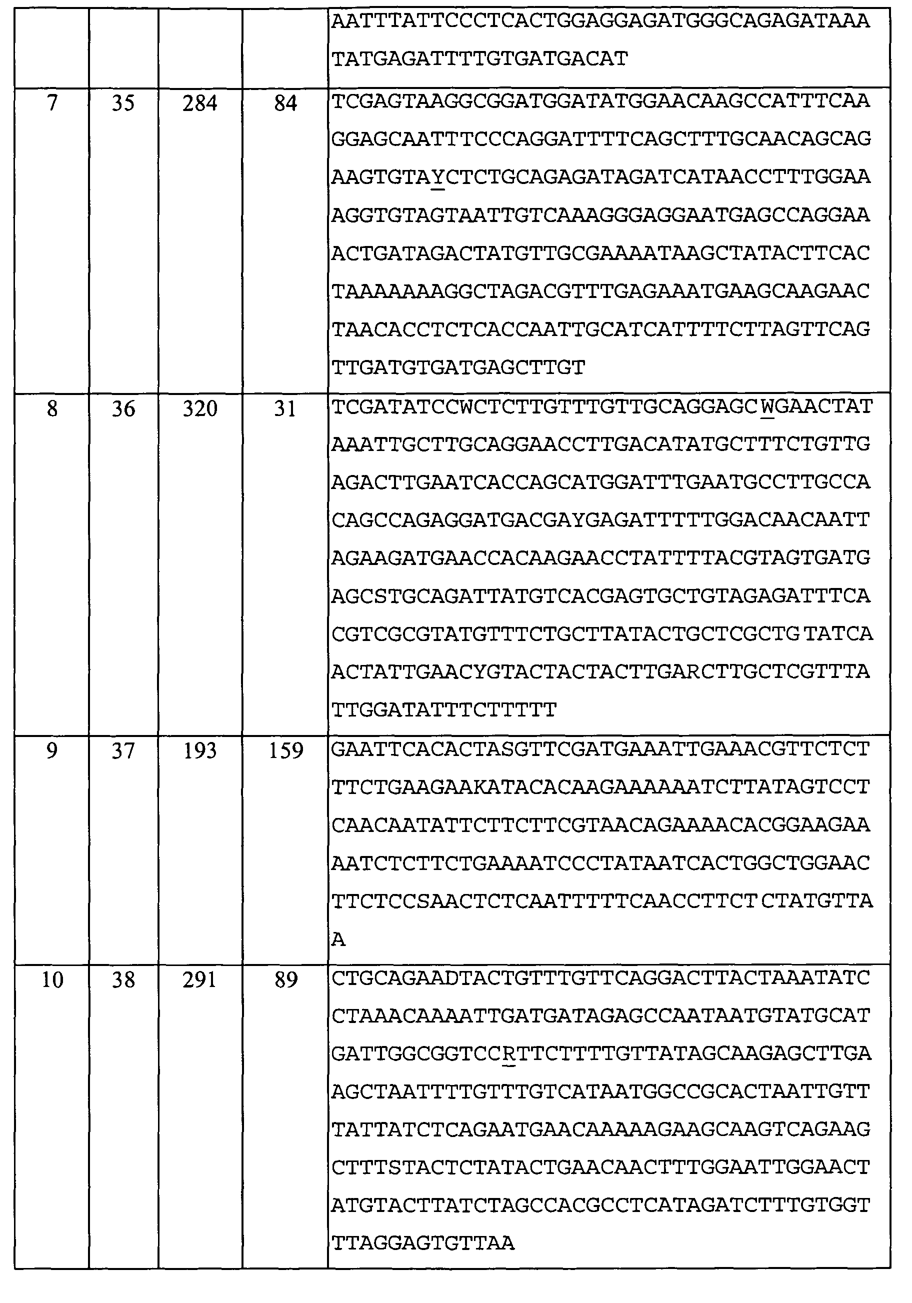

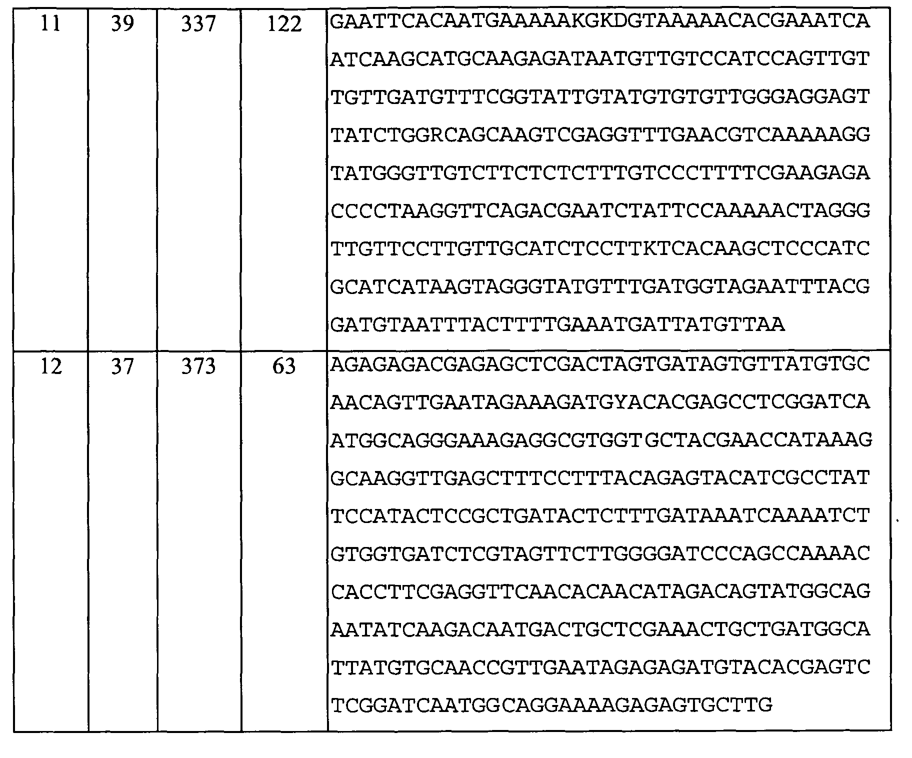

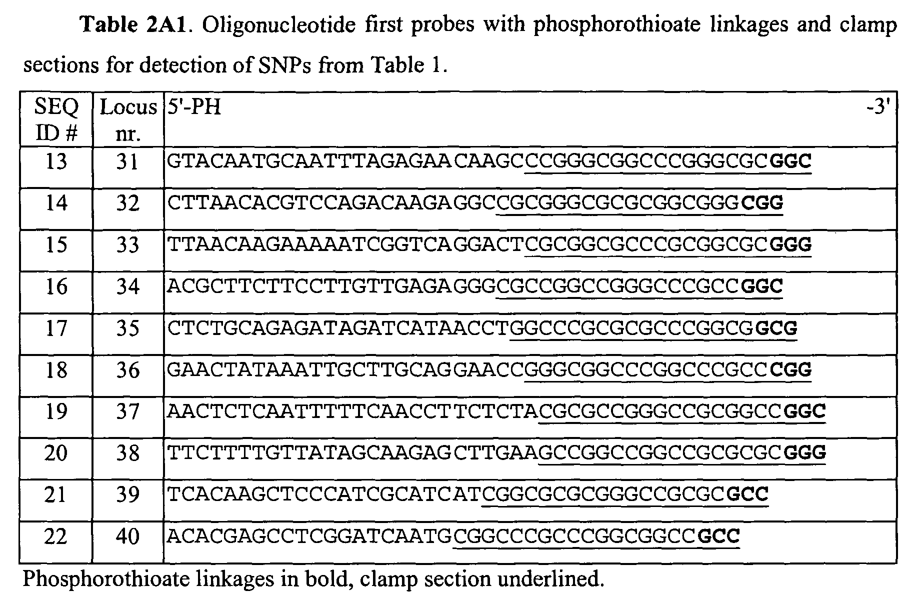

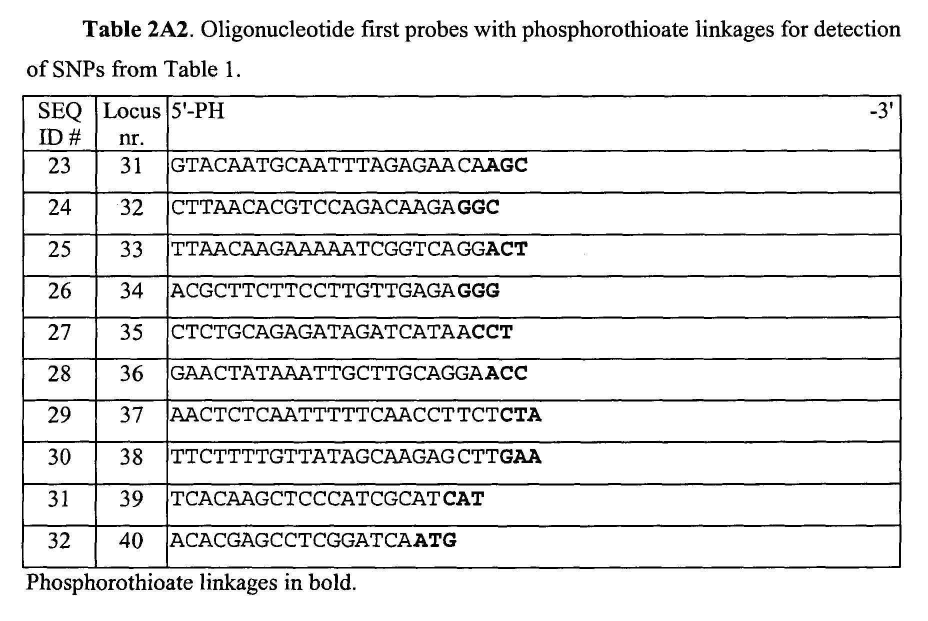

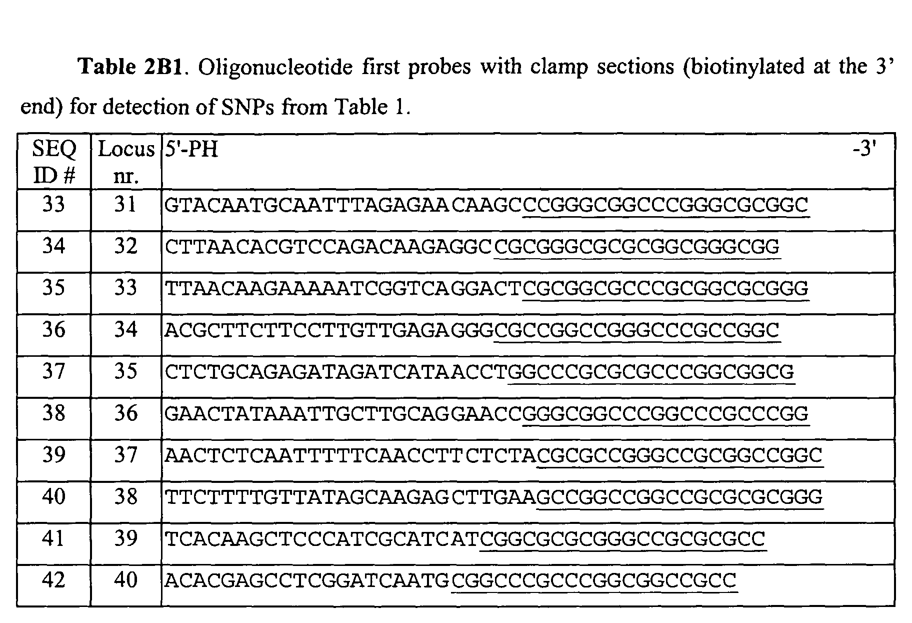

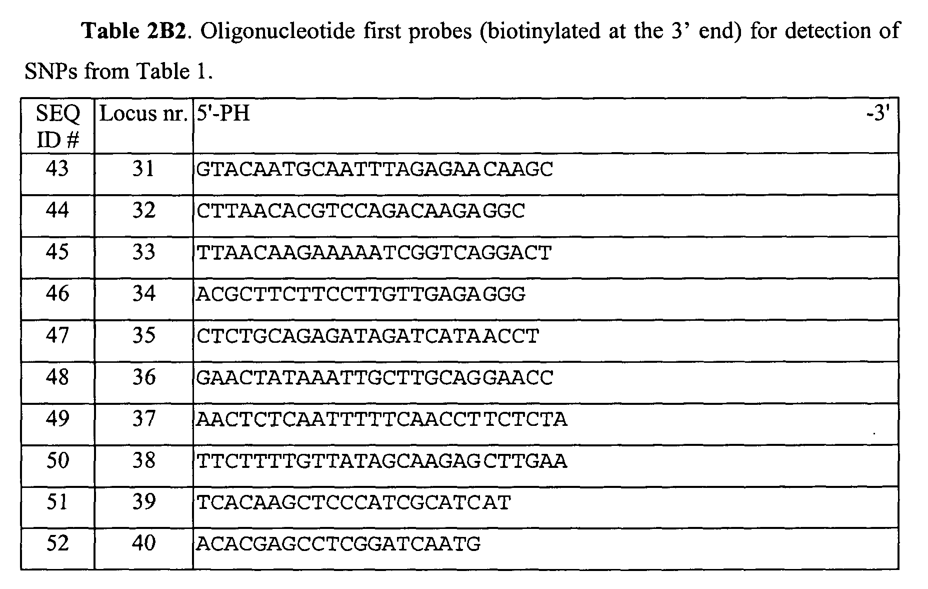

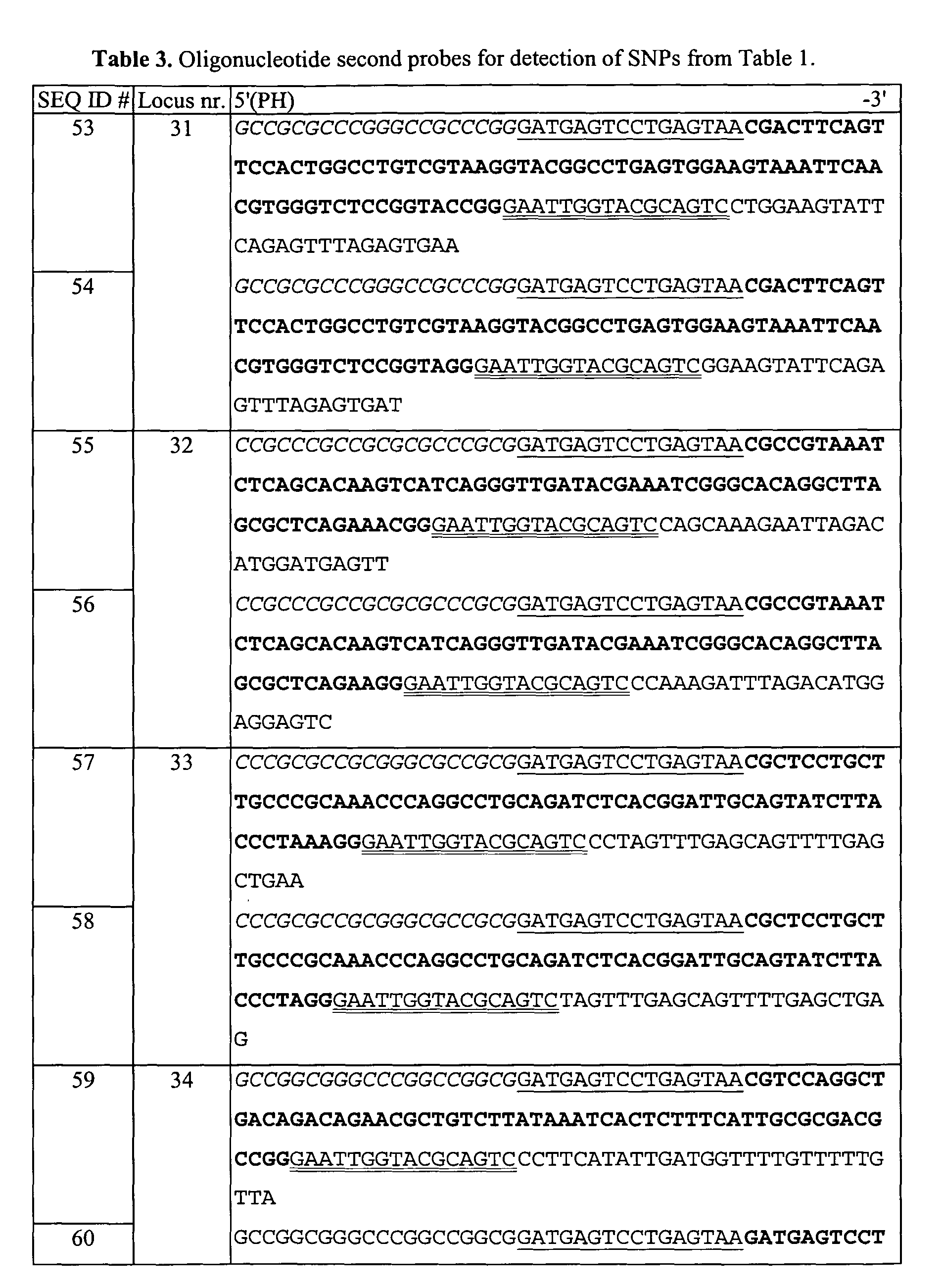

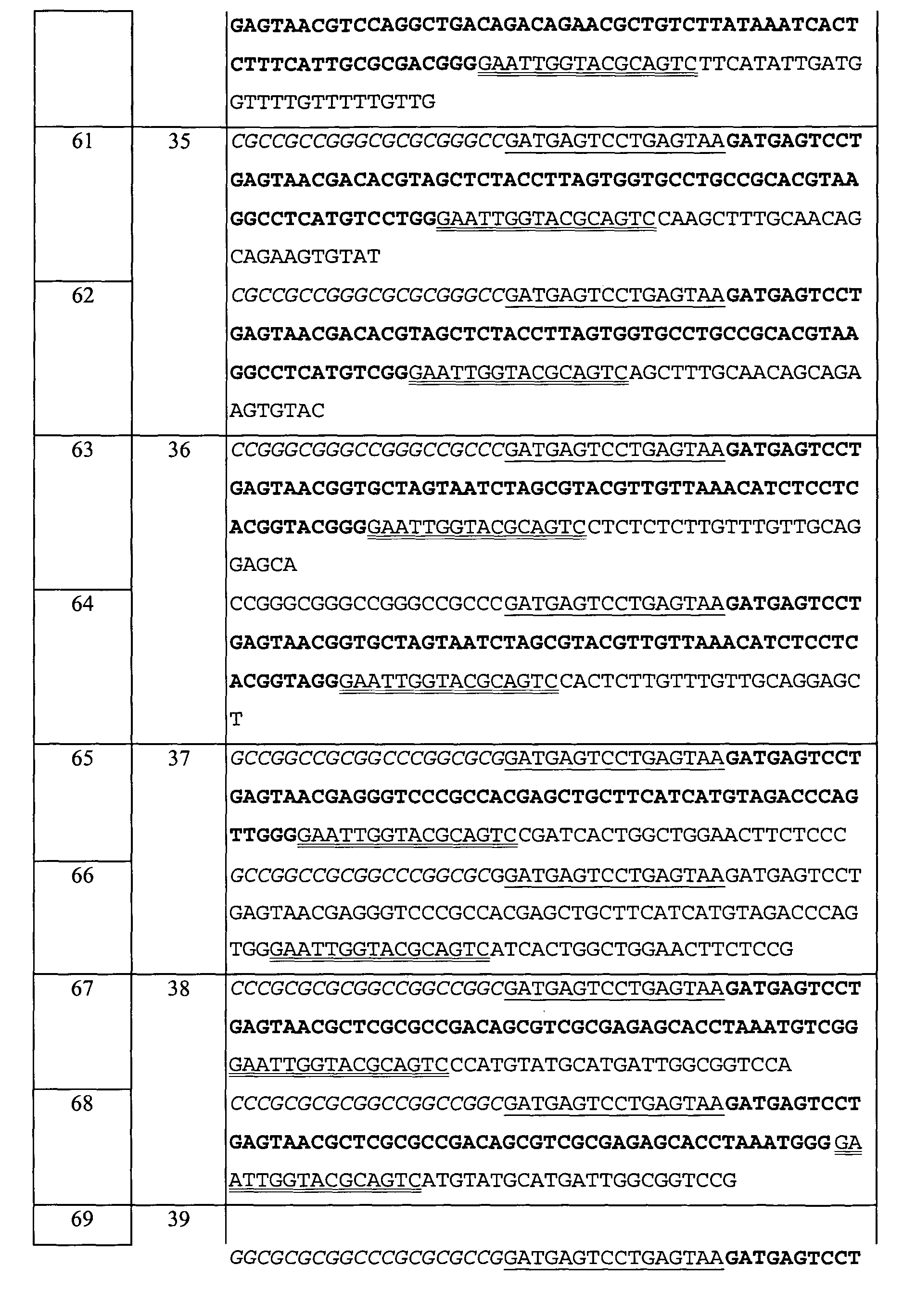

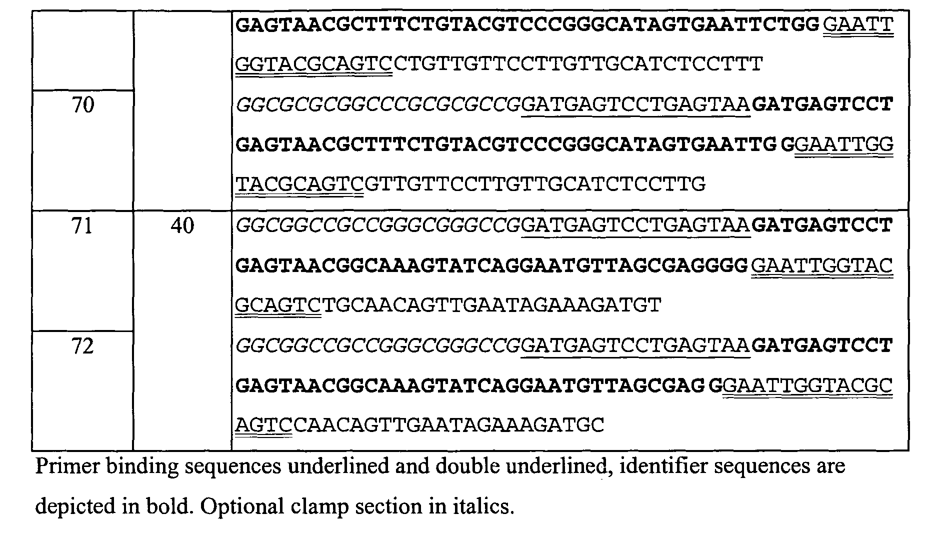

## Claims

1. A method for determining the presence, absence or amount of at least one target nucleic acid sequence in a sample, wherein the method comprises the steps of:
a) providing to the sample a pair of probes for each target nucleic acid sequence to be detected in the sample, wherein the pair comprises at least one first probe comprising a first target-specific section that is complementary to a first part of the target nucleic acid sequence, and at least one second probe comprising a second target-specific section that is complementary to a second part of the target nucleic acid sequence, wherein the probes in each pair are suitable for ligation together when hybridized adjacent to one another on the target nucleic acid sequence, and whereby the second probe further comprises a tag section that is essentially non-complementary to the target nucleic acid sequence, whereby the tag section comprises a first primer-binding sequence, optionally an identifier sequence and optionally a second primer-binding sequence, wherein the first primer binding sequence is located between the second target-specific section and the identifier;
b) allowing the first and second target-specific sections of the first and second probe to anneal to the respective first and second parts of the target nucleic acid sequence;
c) providing means for connecting the first and second target specific sections annealed adjacently to the target nucleic acid sequence;
d) allowing the first and second target specific sections to be connected, to produce a connected probe corresponding to the target sequence in the sample;
e) removing first and/or second probes that have not been connected;
f) optionally, isolating/purifying/separating any non-connected probes and/or connected probes;
g) providing a first primer that is complementary to the first primer-binding sequence;
h) optionally, providing a second primer that is complementary to the second primer-binding sequence;
i) providing a polymerase enzyme and amplifying the resulting mixture of step g) or optionally step h), by elongation of the primer(s) to produce an amplified sample comprising amplicons that are representations of the connected probes;
j) determining the presence, absence or amount of the target sequence in the sample by detecting the presence, absence or amount of the corresponding amplicon.

2. Method according to claim 1, wherein (part of) the first probe and/or the second probe is exonuclease resistant, preferably the first probe.

3. Method according to claim 1 or 2, wherein the first probe is rendered exonuclease resistant at a position distal from the end of the first probe that is annealed adjacent to the end of the target specific section of the second probe.

4. Method according to claims 1-3, wherein the second probe is rendered exonuclease resistant at a position distal from the end of the second probe that is annealed adjacent to the end of the target specific section of the first probe.

5. Method according to any of the claims 1-4, wherein in step (e) the non-connected probes are removed by exonuclease treatment of the sample.

6. Method according to any of the claims 1-5, wherein the exonuclease has 3'→5' activity and/or 5'→ 3' activity.

7. Method according to any of the claims 1-6, wherein the exonuclease is capable of digesting ssDNA and/or *ds*DNA, preferably ssDNA.

8. Method according to any of the claims 1-7, wherein the exonuclease is selected from the group Exonuclease I, Exonuclease III, Exonuclease V, Exonuclease IV, Lambda exonucleases, T7 Exonuclease, strandase exonuclease , 3'-5' Exophosphodiesterases.

9. Method according to any of the claims 1-8, wherein the exonuclease is Exonuclease I or Exonuclease III.

10. Method according to any of the claims 1-9, wherein the first probe contains an affinity ligand, preferably a (strept)avidin-biotin combination.

11. Method according to claim 10, wherein in step (f) the connected probes are isolated/purified/separated from the sample using the affinity ligand.

12. Method according to any of the claims 1-11, wherein the tag section comprises a second primer binding site and wherein the identifier is located between the first and second primer binding site.

13. Method according to any of the claims 1-12, wherein the first or the second probe comprises a further region that is not capable of annealing to the target nucleic acid sequence, which further region is located at the end of the first or second probe at the position of the junction site between the first and second sections of the target nucleic acid sequence.

14. Method according to claim 13, wherein the further region is capable of creating a cleavage structure and whereby exposing the cleavage structure to a cleavage agent will result in cleavage of the cleavage structure when the cleavage structure and cleavage agent are incubated under conditions wherein cleavage can occur.

15. Method according to claims 1-14, wherein at least one of the first or the second primer is a selective primer comprising at least one selective nucleotide at its 3' end.

16. Method according to claims 1-15, wherein an amplicon corresponding to a target sequence in the sample differs from an amplicon corresponding to different target sequence in the sample.

17. Method according to claims 1-16, wherein the tag section comprises an identifier sequence.

18. Method according to claims 1-17, wherein for each amplicon corresponding to a different target sequence in the sample a different identifier is provided

19. Method according to claims 1-18, wherein the presence, absence or amount of a target sequence in a sample is detected by detecting the amplicon representing the connected probe based on molecular mass, length, or sequence.

20. Method according to claims 1-19, wherein the identifier provides the difference in molecular mass, length or sequence.

21. Method according to any of the preceding claims wherein the target sequence is selected from the group of DNA, RNA, polyA⁺ RNA, cDNA, genomic DNA, organellar DNA such as mitochondrial or chloroplast DNA, synthetic nucleic acids, DNA libraries, clone banks or any selection or combinations thereof.

22. Use of a method as defined in any of claims 1-21, for detection of a multiplicity of target nucleotide sequences.

23. Use according to claim 22, for the detection of polymorphisms, preferably single nucleotide polymorphisms.

24. Use according to claim 22 or 23 for transcript profiling, for the detection of the quantitative abundance of target nucleic acid sequences, for genetic mapping, gene discovery, marker assisted selection, seed quality control, hybrid selection, QTL mapping, bulked segregant analysis, DNA fingerprinting and for disclosing information relating to traits, disease resistance, yield, hybrid vigour, and/or gene function.

25. An oligonucleotide acid probe for use in a method as defined in claims 1-21.

26. A pair of two or more oligonucleotide probes, for use in a method as defined in claims 1-21.

27. Use of a pair of two or more oligonucleotide probes as defined in claim 26 wherein the pair comprises a probe for each allele of a single nucleotide polymorphism.

28. A kit comprising oligonucleotide probes suitable for use in a method as defined in claims 1-21.

29. A kit comprising primers for use in a method as defined in claims 1-21.

30. A kit comprising primers and oligonucleotide probes for use in a method as defined in claims 1-21.
